# EUROPEAN PATENT SPECIFICATION

(11) **EP 3 952 873 B1**
(45) Date of publication and mention of the grant of the patent: **30.07.2025**
(21) Application number: 20702191.6
(22) Date of filing: 21.01.2020
(51) Int. Cl.: C07D 487/04, A61K 31/5025, A61P 25/00

(54) **A CLASS OF SMALL-MOLECULE COMPOUNDS AND THEIR PRODRUGS FOR INTERVENTION WITH CAMK2A, AND THEIR USE IN ACUTE BRAIN INJURIES AND CENTRAL HYPERSOMNIAS**
KLASSE VON KLEINMOLEKÜLIGEN VERBINDUNGEN UND DEREN PRODRUGS ZUM EINGRIFF MIT CAMK2A UND DEREN VERWENDUNG IN AKUTEN HIRNVERLETZUNGEN UND ZENTRALEN HYPERSOMNIEN
CLASSE DE COMPOSÉS À PETITES MOLÉCULES ET LEURS PROMÉDICAMENTS POUR UNE INTERVENTION AVEC CAMK2A, ET LEUR UTILISATION EN CAS DE LÉSIONS CÉRÉBRALES AIGUËS ET D'HYPERSOMNIES CENTRALES

(30) Priority: 21.01.2019 DK PA201970040
(43) Date of publication of application: 16.02.2022
(73) Proprietor: Københavns Universitet, 1165 København K (DK)
(72) Inventor: FRØLUND, Bente, 2920 Charlottenlund (DK); WELLENDORPH, Petrine, 2800 Kgs. Lyngby (DK); KORNUM, Birgitte Rahbek, 1855 Frederiksberg C (DK)
(74) Representative: Høiberg P/S
(86) International application number: PCT/DK2020/050022
(87) International publication number: WO 2020/151795

(56) References cited:
- KRALL J ET AL: "Discovery of 2-imidazo[1,2b]pyridazin-2-acetic acid as a new class of ligands selective for GHB high affinity binding sites", JOURNAL OF MEDICINAL CHEMISTRY,, vol. 62, 14 February 2019 (2019-02-14), pages 2798 - 2813, XP002798268
- STN REGISTRY ET AL: "imidazo(1,2-b)pyrdazine-2-acetic acid", AURORA FINE CHEMICALS, 4 May 2016 (2016-05-04), pages 1 - 1, XP093076008, Retrieved from the Internet <URL:www.stn.org> [retrieved on 20230824]
- DATABASE REGISTRY [online] CHEMICAL ABSTRACTS SERVICE, COLUMBUS, OHIO, US; 5 May 2016 (2016-05-05), CHEMICAL CATALOG: AURORA FINE CHEMICALS: "6-(4-methylphenyl)-imidazo[1,2b]pyridazine-2-acetic acid", XP002798263, Database accession no. 1904119-18-1
- DATABASE REGISTRY [online] CHEMICAL ABSTRACTS SERVICE, COLUMBUS, OHIO, US; 5 May 2016 (2016-05-05), CHEMICAL CATALOG: AURORA FINE CHEMICALS: "6-(3-bromophenyl)-imidazo [1,2b]-pyridazine-2-acetic acid", XP002798264, Database accession no. 1904033-16-4
- DATABASE REGISTRY [online] CHEMICAL ABSTRACTS SERVICE, COLUMBUS, OHIO, US; 5 May 2016 (2016-05-05), CHEMICAL CATALOG: AURORA FINE CHEMICALS: "6-(4-pyridinyl)-imidazo [1,2b]pyridazine 2- acetic acid", XP002798265, Database accession no. 1904033-10-8
- DATABASE REGISTRY [online] CHEMICAL ABSTRACTS SERVICE, COLUMBUS, OHIO, US; 5 May 2016 (2016-05-05), CHEMICAL CATALOG: AURORA FINE CHEMICALS: "6-(4-bromophenyl)-imidazo [1,2b]-pyridazine-2-acetic acid", XP002798266, Database accession no. 1903838-62-9
- DATABASE REGISTRY [online] CHEMICAL ABSTRACTS SERVICE, COLUMBUS, OHIO, US; 5 May 2016 (2016-05-05), CHEMICAL CATALOG: AURORA FINE CHEMICALS: "6-(3-methylphenyl)-imidazo [1,2b] pyridazine-2-acetic acid", XP002798267, Database accession no. 1903539-89-8
- J. KRALL ET AL.,: "Discovery of 2-imidazo[1,2b]pyridazin-2-acetic acid as a new class of ligands selective for GHB high affinity binding sites", JOURNAL OF MEDICINAL CHEMISTRY, vol. 62, 14 February 2019 (2019-02-14), pages 2798 - 2813, XP002798268
- SYNTHESIS AND STRUCTURE-ACTIVITY RELATIONSHIPS OF 1-AMINOPHTHALAZINIUM SALTS AS GABA RECPETOR ANATAGONISTS, EUR.J.MED.CHEM., vol. 29, 1 January 1994 (1994-01-01), pages 95 - 105, XP002798269
- LURASCHI E ET AL: "Research on heterocyclic compounds. XXXIII--Synthesis and analgesic activity of imidazo[1,2-b]pyridazine-2-acetic acid derivatives", IL FARMACO, ELSEVIER FRANCE * EDITIONS SCIENTIFIQUES ET MEDICALES, FR, vol. 50, no. 5, 1 May 1995 (1995-05-01), pages 349 - 354, XP009519330, ISSN: 0014-827X
- LEBEK S ET AL: "The novel CaMKII inhibitor GS-680 reduces diastolic SR Ca leak and prevents CaMKII-dependent pro-arrhythmic activity", JOURNAL OF MOLECULAR AND CELLULAR CARDIOLOGY, ACADEMIC PRESS, GB, vol. 118, 31 March 2018 (2018-03-31), pages 159 - 168, XP085396153, ISSN: 0022-2828, DOI: 10.1016/J.YJMCC.2018.03.020

## Description

### Field of the invention

The present invention relates to small-molecule compounds that are suitable for use in treatment of neurological conditions where Ca²⁺/calmodulin-dependent protein kinase 2a (CaMK2a) is involved.

### Introduction to the invention

γ-hydroxybutyric acid (GHB) is present in the brain in micromolar concentrations and binds with high affinity to a specific protein (Bay et al. 2014), recently identified as CaMK2a, one of the most abundant proteins in the postsynaptic density. CaMK2a is a major regulator of synaptic signaling through its phosphorylation of ion channels and neurotransmitter receptors and is intimately involved in long-term potentiation and synaptic plasticity, and thus higher brain functions such as learning and memory (Hell, 2014).

### Current state of the art

Due to its central role in regulating synaptic function, CaMK2a is involved in most neurodegenerative diseases and a promising drug target, yet unexplored due to the unavailability of small-molecule brain-penetrant ligands with selectivity for the 2a subtype.

CaMK2a is modulated by an inhibitory peptide Tat-CN21, which has shown promise in post-stroke treatment in preclinical studies (Coultrap et al., 2011). In addition to potential issues with stability and cell penetrance as well as bioavailability related to general use of peptides, Tat-peptides are further known to cause hypertension. From data obtained by the inventors, GHB and related small-molecule analogues bind directly to CaMK2a, but to a site distinct from CN21.

GHB is highly efficacious in treating cataplexy and excessive daytime sleepiness in relation to narcolepsy and shows better efficacy than the GABA_{B} selective agonist baclofen. Specifically, GHB uniquely works on daytime symptoms (Huang and Guilleminault, 2009). Compounds related to GHB may thus have efficacy in narcolepsy through effects on CaMK2a and/or GABA_{B} receptors.

GHB is currently the best treatment for cataplexy.

The inventors have previously published two series of selective ligands with mid to high nanomolar affinity for GHB binding sites i.e. CaMK2a: i) The conformationally restricted

GHB analogue 3-hydroxycyclopent-1-enecarboxylic acid (HOCPCA), displaying 27 times higher affinity than GHB, and ii) the 4-substituted biaromatic GHB analogues, represented by 4-(4-benzyloxy)phenyl)-4-hydroxybutanoic acid (BnOPh-GHB), displaying 39 times improved affinity compared to GHB (Bay et al., 2014). Overall, the chemical diversity of ligands for the high-affinity GHB binding site is sparse and, in essence, only represented by the core structures of GHB, HOCPCA and (*E*)-2-(5-hydroxy-5,7,8,9-tetrahydro-6*H*-benzo[7]annulen-6-ylidene)acetic acid (NCS-382). GHB, HOCPCA and an analogue of NCS-382 have been shown to work in mouse model of acute stroke. Additional development of novel small-molecule chemical entities is needed to advance and explore CaMK2a as a drug target in brain injuries and central hypersomnias.

Colotta et al. 1994 (Eur J Med Chem; 29, 95-105, discloses the synthesis and SAR studies of 1-aminophthalazinium salts as GABA_{A} receptor antagonists. Lurashi et al. 1995 (Farmaco, 50(5), 349-354) discloses the synthesis and analgesic activity of imid-azo[1,2-*b*]pyridazine-2-acetic acid derivatives. Lebek et al. 2018 (J Mol Cell Cardio, 118, 159-168) discloses a study of the effect of a CAMKII inhibitor GS-680 on diastolic SR Ca leak and CaMKII-dependent pro-arrhythmic activity.

### Description of the invention

γ-Hydroxybutyric acid (GHB) is a neuromodulator, present in micromolar concentration in the mammalian brain as a metabolite of γ-aminobutyric acid (GABA). GHB is used clinically as a prescribed drug in narcolepsy, and as a recreational drug (e.g. Fantasy). GHB displays low affinity (millimolar) binding to the GABA_{B} receptor and high-affinity (nano- to micromolar) to an abundant highly specific protein (Bay et al., 2014), recently identified to be CaMK2a.

The invention is defined by the claims. Any subject matter falling outside the scope of the claims is provided for information purposes only. The references to methods of treatment in this description are to be interpreted as references to the compounds, pharmaceutical compositions and medicaments of the present invention for use in a method for the treatment of the human (or animal) body by therapy (or for diagnosis).

Until recently now, no small-molecule ligands for selectively targeting CaMK2a have been described. A specific class of GHB-related analogues, herein represented by JON-11 (termed JON-11 analogues) are first-in-class compounds with promise to treat conditions associated with CaMK2a malfunction. In one setting, compounds may be neuroprotective after an acute insult or after lack of oxygen to the brain. In another setting, compounds may improve symptoms associated with sleep disturbances and cataplexies, e.g. in the sleep disorder narcolepsy. Effects may be mediated through effects on CaMK2a autophosphorylation, substrate phosphorylation or kinase activity in general.

As CaMK2a is an intracellular protein, compounds must pass the plasma membrane to act. Being carboxylic acids, GHB ligands are charged at physiological pH. As a consequence, the compounds as such depend on active transport to enter the brain or neurons. At the blood-brain barrier, this is governed by the monocarboxylate transporter 1 where HOCPCA and GHB are substrates (Thiesen et al., 2015). Neurons express MCT1 and 2, supposedly mediating the uptake here. Another way to promote brain penetration is through the attachment of a chemical pro-moiety so as to promote passive diffusion or target specific transporters involved in brain uptake (e.g. the large amino acid transporter (LAT1)) (Puris et al., 2017).

The invention provides compounds having the following general formula I: 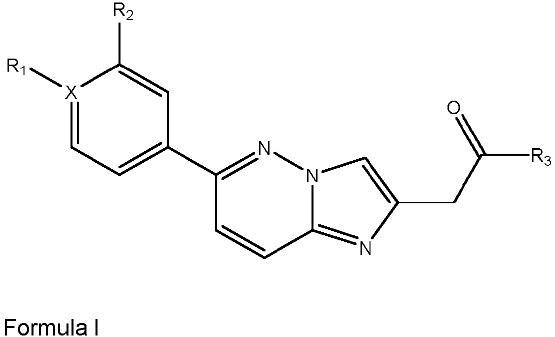 wherein X is C, R₁ and R₂ are different and selected from H, F, I, Cl, -OH, - NH₂, ethyl, propyl, isopropyl, butyl, isobutyl, *tert*-butyl, pentyl, hexyl wherein butyl, pentyl and hexyl are linear or branched, -O-C₁-C₆-alkyl, -O-benzyl (-O-Bn), wherein C₁-C₆-alkyl is linear or branched and wherein benzyl is unsubstituted or substituted with one or more halogen and/or one or more -C₁-C₆-alkyl or -O-C₁-C₆-alkyl;
or
X is C, R₁ and R₂ are the same and selected from F, I, Cl, -OH, -NH₂, ethyl, propyl, isopropyl, butyl, isobutyl, *tert*-butyl, pentyl, hexyl wherein butyl, pentyl and hexyl are linear or branched, -O- C₁-C₆-alkyl, -O-benzyl (-O-Bn), wherein C₁-C₆-alkyl is linear or branched and wherein benzyl is unsubstituted or substituted with one or more halogen and/or one or more C₁-C₆-alkyl or -O-C₁-C₆-alkyl;
and
R₃ is selected from -OH, -NH₂, -O-C₁-C₆-alkyl, -C₁-C₆-alkyl-O-C(=O)-C₁-C₆-alkyl, -NH-C₁-C₆-alkyl, -NH-aryl, -NH-substituted aryl, wherein C₁-C₆-alkyl is linear or branched, aryl is unsubstituted or substituted with one or more amino acid residues to form a compound in the following formula II illustrated with a substituted aryl being represented by phenylalanine, but any other amino acid residue is also within the scope of the present invention: 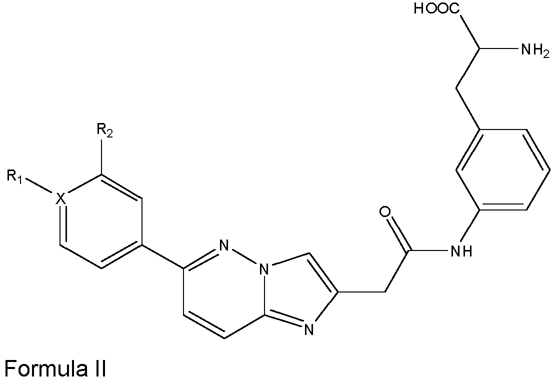
Halogen is selected from F, I, Cl and Br;
or pharmaceutically acceptable salts thereof.

In an alternative embodiment, the invention relates to compounds of formula I, wherein:
X is C, R₁ and R₂ are different and selected from H, F, I, Cl, -OH, -NH₂, -NO₂, ethyl, propyl, isopropyl, butyl, isobutyl, *tert*-butyl, pentyl, hexyl wherein butyl, pentyl and hexyl are linear or branched, -C₁-C₆-alkyl, -O-C₁-C₆-alkyl, -O-benzyl (-O-Bn), wherein C₁-C₆-alkyl is linear or branched and wherein benzyl is unsubstituted or substituted with one or more halogen and/or one or more -C₁-C₆-alkyl or -O-C₁-Cₑ-alkyl;
   or
X is C, R₁ and R₂ are the same and selected from F, I, Cl, -OH, -NH₂, -NO₂, ethyl, propyl, isopropyl, butyl, isobutyl, *tert-*butyl*,* pentyl, hexyl wherein butyl, pentyl and hexyl are linear or branched, -O- C₁-C₆-alkyl, -O-benzyl (-O-Bn), wherein C₁-C₆-alkyl is linear or branched and wherein benzyl is unsubstituted or substituted with one or more halogen and/or one or more C₁-C₆-alkyl or -O-C₁-C₆-alkyl;
   and
   R₃ is selected from -OH, -NH₂, -O-C₁-C₆-alkyl, -C₁-C₆-alkyl-O-C(=O)-C₁-C₆-alkyl, -O-aryl, -O-substituted aryl, -NH-C₁-C₆-alkyl, -NH-aryl, -NH-substituted aryl, wherein C₁-C₆-alkyl is linear or branched, the substituted aryl is substituted with one or more amino acid residues. When R₃ is either an -O-substituted aryl or a -N-substituted aryl, R₃ is preferably selected from the following:

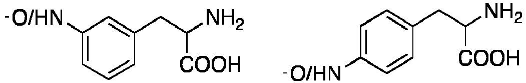

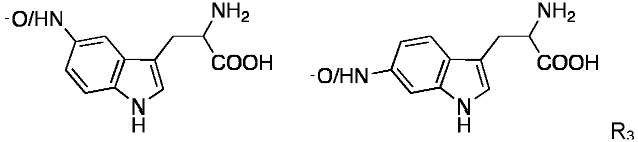

The invention also relates to the compounds mentioned herein in any form including geometric isomers, tautomers, enantiomers, racemic mixtures, or deuterated derivatives.

The pharmaceutically acceptable salts include salts of organic or inorganic acids or salts of bases. Specific examples are e.g. salt like hydrobromide, hydrochloride, trifluro-acetate or salts like alkali salts, e.g. sodium or lithium or alkali earth metal salts. C₁-C₆-alkyl is selected from methyl, ethyl, propyl, isopropyl, butyl, isobutyl, tert.butyl, pentyl, pentyl, hexyl; butyl, pentyl and hexyl may be linear or branched.

Specifically, the present invention provides novel compounds according to formula I, wherein R₁ and R₂ are different and selected from H, F, I, Cl, -O-C₁-C₆-alkyl and -O-Bn, and R₃ and alkyl being as defined above. Specifically, the present invention provides novel compounds according to formula I, wherein R₁ and R₂ are the same and selected from F, I, Cl, -O-C₁-C₆-alkyl and -O-Bn, and R₃ and alkyl being as defined above.

The present invention also provides novel compounds according to formula I, wherein R₁ and R₂ are different and selected from H, F, I, Cl, -O-C₁-C₆-alkyl and -O-Bn , and R₃ is selected from -OH, -O-C₁-C₆-alkyl, -C₁-C₆-alkyl-O-C(=O)-C₁-C₆-alkyl, and -NH-substituted aryl, wherein alkyl and aryl are as defined above. The present invention also provides novel compounds according to formula I, wherein R₁ and R₂ are the same and selected from F, I, Cl, -O-C₁-C₆-alkyl and -O-Bn , and R₃ is selected from -OH, -O-C₁-C₆-alkyl, -C₁-C₆-alkyl-O-C(=O)-C₁-C₆-alkyl, and -NH-substituted aryl, wherein alkyl and aryl are as defined above.

Compounds of the present invention are also compounds according to formula I, wherein:
one of R₁ and R₂ is H and the other is selected from F, I, Cl, -O-C₁-C₆-alkyl and -O-Bn;
or wherein R₁ and R₂ are different and selected from F, I, Cl, -O-C₁-C₆-alkyl;
or wherein R₁ and R₂ are the same and selected from F, I, Cl, -O-C₁-C₆-alkyl
and -O-Bn, and R₃ is as defined above;
and -O-Bn, and R₃ is as defined above.

Compounds of particular interest are those wherein X is C.

Within the scope of the present invention are compounds, wherein R₃ is selected from - NH₂, -O-C₁-C₆-alkyl, -C₁-C₆-alkyl- O-C(=O)-C₁-C₆-alkyl, -NH-C₁-C₆-alkyl, -NH-aryl, -NH-substituted aryl, wherein -C₁-C₆-alkyl islinear or branched, aryl may be unsubstituted or substituted with one or more amino acid residues to form a compound in the following formula II illustrated with a substituted aryl being represented by phenylalanine, but any other amino acid residue is also within the scope of the present invention; i.e. compounds wherein R₃ is not -OH.

In another aspect, the present invention relates to compounds of formula I, where X is C, R₁ and R₂ are different and is either H or -OH, and R₃ is OH. In another aspect, the present invention relates to compounds of formula I, where X is C, R₁ and R₂ are the same and is -OH, and R₃ is OH.

In a further aspect, the invention relates to compounds of formula I, where X is C, R₁ and R₂ are different and selected from H, -OH, F, I, Cl, NO₂ and -O-Bn, and R₃ is selected from -O-C₁-C₆-alkyl, -C₁-C₆-alkyl- O-C(=O)-C₁-C₆-alkyl, -NH-C₁-C₆-alkyl. In a further aspect, the invention relates to compounds of formula I, where X is C, R₁ and R₂ are the same and selected from -OH, F, I, Cl, NO₂ and -O-Bn, and R₃ is selected from - O-C₁-C₆-alkyl, -C₁-C₆-alkyl- O-C(=O)-C₁-C₆-alkyl, -NH-C₁-C₆-alkyl.

In an even further aspect, the invention relates to compounds of formula I, where:
X is C, R₁ and R₂ are different and selected from H, -OH, F, I, Cl, NO₂ and -O-Bn,
   or
X is C, R₁ and R₂ are the same and selected from -OH, F, I, Cl, NO₂ and -O-Bn,
   and R₃ is selected from:

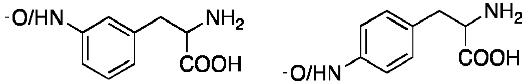

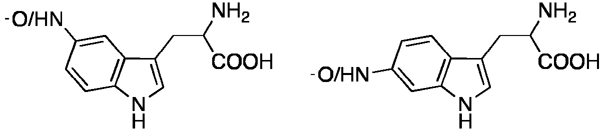

Compounds disclaimed from the present invention and known from a previous publication are compounds having formula I, wherein:

| X | R₁ | R₂ | R₃ |
|---|---|---|---|
| C | Cl | H | OH |
| C | H | Cl | OH |
| C | OMe | H | OH |
| C | H | OMe | OH |
| C | Cl | Cl | OH |
| C | OBn | H | OH |
| C | H | OBn | OH |

or hydrochloride or TFA salts thereof.

Specific examples of compounds of the invention or for use in accordance with the present invention are given in the examples herein.

The present invention discloses a novel chemical scaffold of formula I (representing inter alia JON-11 (example 1) and the compounds exemplified herein) with affinity for CaMK2a as shown in binding assays to native and recombinant CaMK2a. Furthermore, it is contemplated that the compounds, wherein R₃ is -OH or -NH₂ are therapeutically active, but there may be compounds that are not capable of entering the brain. For such compounds, the R3 group may be changed to a pro-moiety such as e.g. an ester.

The pro-moiety makes it possible for the compound to enter the brain either passively, or facilitated by a transporter such as LAT1, in which the pro-moiety is cleaved off leaving the therapeutically active compound within the brain. Such prodrugs are also part of the invention and within the scope of formula I and thus part of the present invention as defined in the claims.

As shown in the examples, the present invention provides an ester prodrug (pro-JON-11 (10); example 10, a prodrug of JON-11) which after systemic administration displays good plasma stability, freely enters the brain and is cleaved to the parent drug. This is in contrast to JON-11 which is not brain-permeable after intraperitoneal (i.p.) injection. JON-11 displays only weak affinity for GABA_{A} receptors, which may be of interest in conditions with an aberrant GABA_{A} receptor activity.

The physico-chemical properties of pro-JON-11, entrance into the brain, and binding to the target make small molecules of this formula vastly interesting. It is contemplated that the compounds of formula (I) also have acceptable physico-chemical properties. Given that no CaMK2a small-molecule non-peptide selective ligands exist, they may also represent a novel mechanism of action.

The inventors herein demonstrate that JON-11 and novel analogues bind with high affinity to CaMK2a and can be delivered to the brain using the prodrug strategy.

The compounds of formula I may be synthesized in accordance with the following schemes:

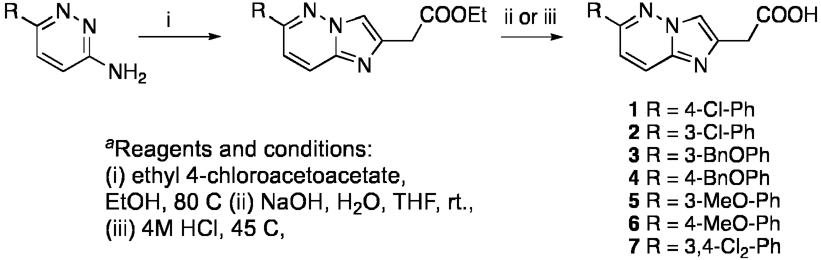

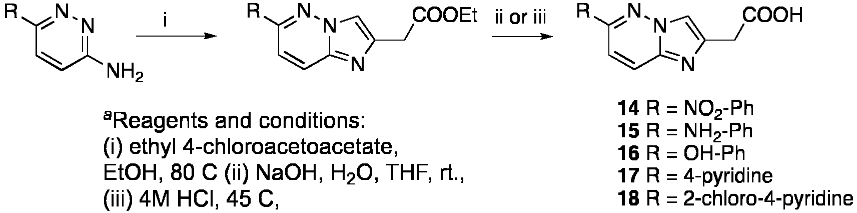

### Use of the compounds of the invention

In this specific aspect of the invention, all compounds are contemplated to be useful in the diseases/conditions mentioned in the following; i.e. also the compounds mentioned in the table above. However, some of the compounds wherein R₃ is -OH may not reach the brain, but are active if delivered to the brain. If this is the case, the -OH group is changed to one of the other R₃ groups mentioned herein so that they contain a pro-moiety that enables transport over the brain barrier and which - after entering the brain - is cleaved off.

One embodiment provides for a compound of general formula I 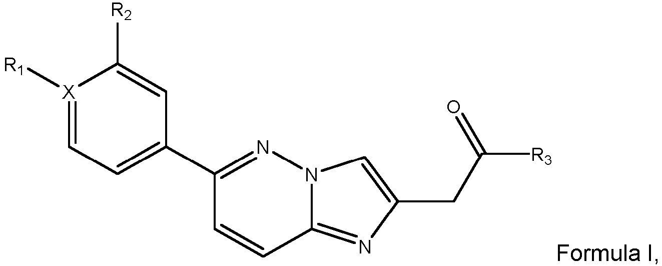 or a pharmaceutically acceptable salt thereof,
wherein X is C, R₁ and R₂ are the same or different and selected from H, halogen, -OH, -NH₂, -NO₂, -C₁-C₆-alkyl, -O-C₁-C₆-alkyl, O-benzyl (O-Bn), wherein C₁-C₆-alkyl is linear or branched and wherein benzyl is unsubstituted or substituted with one or more halogen and/or one or more -C₁-C₆-alkyl or -O C₁-C₆-alkyl; when X is N then R₁ is absent; and
R₃ is selected from -OH, -NH₂, -O-C₁-C₆-alkyl, -C₁-C₆-alkyl-O-C(=O)-C₁-C₆-alkyl, -NH-C₁-C₆-alkyl, - O-aryl, -O-substituted aryl -NH-aryl, -NH-substituted aryl, wherein C₁-C₆-alkyl is linear or branched, aryl is unsubstituted or substituted with one or more amino acid residues to form a compound in formula II illustrated with a substituted aryl being represented by phenylalanine: 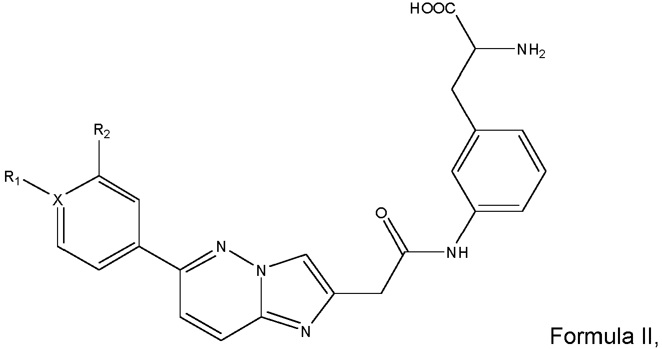
or a pharmaceutically acceptable salt thereof for use in medicine.

The compounds of the invention can be used as a medicament for treating diseases. Thus, the invention also relates to compounds of formula I for use as a medicament.

In one embodiment, the compounds of the present invention are contemplated to be useful in the treatment of acute brain injuries. Brain injuries may be caused by an acute primary cerebral or ischemic insult, e.g. traumatic brain injury, stroke, subarachnoid haemorrhage, neonatal hypoxia-ischemia encephalophathy or associated in utero complications, haemodynamic shock with cardiac arrest, and global hypoperfusion during surgery or as a result from heart failure.

In another embodiment, the compounds are contemplated to have beneficial effects in preventing and/or alleviating central hypersomnias and cataplexies. Central hypersomnias include idiopathic hypersomnia, recurrent hypersomnia such as Klein-Levin syndrome and narcolepsy including with cataplexy (narcolepsy type 1; narcolepsy-cataplexy syndrome; NRCLP1; narcolepsy with low hypocretin) and narcolepsy without cataplexy (narcolepsy type 2; narcolepsy with normal hypocretin).

Narcolepsy Type 1 and Type 2 are sleep disorders characterised by excessive daytime sleepiness and narcolepsy Type 1 is further characterised by cataplexy. Cataplexy is characterised by sudden loss of muscle tone. The duration of cataplexy is usually short, ranging from a few seconds to several minutes and recovery is immediate and complete. The loss of muscle tone varies in severity and ranges from a mild sensation of weakness with head drop, facial sagging, jaw drop, slurred speech and buckling of the knees to complete postural collapse, with a fall to the ground. Cataplexy is usually precipitated by emotion that usually has a pleasant or exciting component, such as laughter, elation, pride, anger or surprise.

Besides excessive daytime sleepiness and cataplexy (in narcolepsy type 1), individuals affected by narcolepsy often present symptoms such as sleep fragmentation, abnormal rapid eye movement sleep, nocturnal sleep disruption, paralysis during sleep onset or during awakening, and/or hypnagogic hallucinations. Similar symptoms are shown also by individuals affected by Narcolepsy Due to Medical Condition (NDMC), a group of disorders also known as secondary or symptomatic narcolepsy.

Examples of medical conditions causing narcolepsy symptoms including cataplexy are: tumors, sarcoidosis, arteriovenous malformations affecting the hypothalamus, multiple sclerosis plaques impairing the hypothalamus, paraneoplastic syndrome antt-Ma2 antibodies, Neimann-Pick type C disease or Coffin-Lowry syndrome. Examples of medical conditions commonly causing narcolepsy symptoms without cataplexy are: head trauma, myotonic dystrophy, Prader-Willi syndrome, Parkinson's disease or multisystem atrophy.

Cataplexy is a hallmark of narcolepsy but may also be associated with specific lesions located primarily in the lateral and posterior hypothalamus, as e.g. tumors (astrocytoma, glioblastoma, glioma, craniopharyngioma and subependynoma) and arterio-venous malformations. Conditions in which cataplexy can be seen include ischemic events, multiple sclerosis, head injury, paraneoplastic syndromes, and infections, such as encephalitis. Cataplexy may occur transiently or permanently due to lesions of the hypothalamus that were caused by surgery, especially in difficult tumor resections. In infancy, cataplexy can be seen in association with other neurological syndromes such as Niemann-Pick type C disease.

### Definitions

### Acute brain injury

The term 'acute brain injury' as used herein refers to a primary cerebral or ischemic insult that damages brain tissue in an acute manner, but also initiates cascades of devastating neurotoxic effects. Examples include traumatic brain injury, stroke, subarachnoid haemorrhage, neonatal hypoxia-ischemia encephalopathy or associated in utero complications, haemodynamic shock with cardiac arrest, and global hypoperfusion during surgery or as a result from heart failure.

### Autophosphorylation

The term 'autophosphorylation' as used herein refers to the phosphorylation of CaMK2a on residue Thr286.

### CaMK2a

The term 'CaMK2a' as used herein refers to Calcium/calmodulin-dependent protein kinase type 2 alpha.

### Cataplexy

The term 'cataplexy' is a sudden and transient episode of muscle weakness accompanied by full conscious awareness, typically triggered by emotions such as laughing, crying, or terror.

### Central hypersomnia

Disorders of excessive daytime sleepiness related to the central nervous system, i.e., the brain. These disorders share in common the predominant symptom of daytime sleepiness. Various types of central hypersomnias exist, including idiopathic hypersomnia, recurrent hypersomnia such as Klein-Levin syndrome, and narcolepsy.

### JON-11 analogue

The term 'JON-11 analogue' as used herein refers to a specific class of GHB-related analogues, typified by 2-(6-(4-chlorophenyl)imidazo[1,2-b]pyridazin-2-yl)acetic acid.

### LAT-1

This refers to the L-type amino acid transporter expressed at the blood-brain-barrier.

### Neuroprotective

The term 'neuroprotective' as used herein refers to the ability of a chemical substance to prevent nerve cell damage such as that induced by acute injury to the brain.

### Photothrombotic focal ischemia

The term 'photothrombotic focal ischemia' as used herein refers to method of introducing a cortical infarction through a photochemical reaction with a light-sensitive dye delivered by i.p. injection.

### Prodrug

A pharmacological inactive substance that is the modified form of a pharmacological active compound to which it is converted e.g. by enzymatic action in the body.

### Pro-moiety

A moiety which is linked to a pharmacological active compound to obtain a prodrug.

### Recombinant

The term 'recombinant' as used herein refers to DNA sequences that have been transfected into and expressed as proteins in HEK293T cells.

### Legends to figures

Figure 1: JON-11 and the related analogues **2-7** compete with [³H]NCS-382 for binding to the GHB high-affinity binding site/CaMK2a with affinities in the mid to high nanomolar range.
Figure 2: JON-11 binds directly to recombinant CaMK2a expressed transiently in HEK293T cells and does so with higher affinity than GHB.
Figure 3: JON-11 and calmodulin both bind directly to full-length purified synthetic CaMK2a as demonstrated by surface plasmon resonance (SPR) binding.
Figure 4: Metabolic stability of JON-11 and prodrugs **1a, 8-12, 19** and **20** in mouse liver microsomes (MLM) and mouse plasma (MP).
Figure 5: Plasma and brain exposure of JON-11 and pro-JON-11 (**10**) in male mice after i.p. injection. JON-11 enters the brain to a very low extent (B/P ratio 0.05 in mice, dose 10 mg/kg i.p. injected), whereas pro-JON-11 enters the brain (B/P ratio 2.24, dose 10 mg/kg i.p.).
Figure 6: Lack of agonistic activity of JON-11 at recombinant GABAB receptors in concentrations up to 300 µM.
Figure 7: The JON-11 prodrug **11,** but not **12,** competes with [³H]leucine uptake at LAT-1 but cannot be exchanged for [³H]leucine.
Figure 8: JON-11 (100 mg/kg i.p.) does not affect locomotor activity in mice.
Figure 9: JON-11 significantly decreases the number of cataplexy attacks in a mouse model of narcolepsy after 8-11 days of chronic administration (100 mg/kg i.p.).

### Examples

### Materials and methods

### Rat brain membrane [³H]NCS-382 binding assay

According to previously published protocols, rat (Sprague-Dawley) cortical synaptic membranes (P2 synaptosomes) were prepared and stored at -20 °C until the day of assay (Wellendorph et al., 2005). Membranes were then washed three times with binding buffer (50 mM potassium phosphate buffer pH 6.0). The binding protocol was performed in 96-well microplates. In brief, compounds were incubated with 16 nM [³H]NCS-382 radioligand and membranes for 60 min at 0-4 °C, filtered through GF/C filter plates (PerkinElmer) using a 96-well harvester (Packard), rapidly washed three times with ice-cold binding buffer, and filter plates dried. CPM values were determined using liquid scintillation counting in a TopCount NXT Microplate Scintillation & Luminescence Counter (PerkinElmer). Curves were generated using non-linear regression (GraphPad Prism 7, GraphPad Prism Software, San Diego, CA, USA).

[³H]HOCPCA binding to recombinant CaMK2a expressed in HEK293T cells HEK293T were cultured using standard conditions, using Dulbecco's modified Eagle Medium with GlutaMax, 10% fetal bovine serum and 1% penicillin-streptomycin, and incubated at 37 °C in a humidified atmosphere of 95% O₂ and 5% CO₂. Cells were transfected with cmyc-tagged rat CaMK2a (Origene construct RR201121) using PolyFect (Qiagen, West Sussex, UK) according to the manufacturer's protocol. Whole cell homogenates were prepared 48 hr post-transfection by washing the cells with ice-cold 1x PBS and harvesting by scraping. Cells were collected and centrifuged for 10 min at 1000 x g. Cell pellets were resuspended in ice-cold 1x PBS and homogenized using 2 x 1 mm zirkonium beads in a bullet blender for 20 s at max speed (NextAdvance, NY, USA). Homogenates were cleared by centrifugation (10 min, 4 °C, 14.000 x *g*). Protein concentration was determined using the Bradford protein assay. 150-200 µg proteins were incubated with 5 nM ³H-HOCPCA (Vogensen et al., 2013) and test compound in 1 ml total volume for 1 hr at 0-4 °C. Nonspecific binding was determined with 1-10 mM GHB. Proteins were then precipitated by addition of ice-cold acetone (4x of the assay volume), vortexing and incubation at -20 °C for 1 hr. Proteins were filtered rapidly through GF/C unifilters (Whatman) and washed using a 48-well harvester. The dried filters were added scintillation liquid and radioactivity measured on a Tricarb 2100 Scintillation counter (Packard).

### Surface Plasmon Resonance (SPR) binding studies

To further confirm direct binding of JON-11 to CaMK2a, SPR was used. SPR measurements were performed at 25 °C using a Pioneer FE instrument from PALL FortéBio. Full-length human recombinant CaMK2a with an N-terminal GST fusion protein (Carna Biosciences, number 02-109) was immobilized to a biosensor chip by amine coupling to 2402 response units (RU) using a 20 mM sodium acetate pH 5 immobilization buffer. JON-11 was injected in 2-fold serial dilution (ranging from 2.5-40 µM) over immobilized CaMK2a using a HBS running buffer pH 7.4 (10 mM Hepes, 150 mM NaCl, 0.005% tween 1 mM DTT) supplemented with Ca²⁺ (500 µM). Calmodulin (Sigma) was used as a positive control to evaluate activity of the immobilized protein. Calmodulin was injected in 2-fold serial dilution (ranging from 16 nM-32 µM) over immobilized CaMK2a. Between calmodulin injections, the biosensor chip surface was regenerated by injections of HBS buffer supplemented with 100 µM EDTA. The data were analyzed using Qdat Data Analysis Tool version 2.6.3.0 (PALL FortéBio). The sensorgrams were corrected for buffer bulk effects and unspecific binding of the samples to the chip matrix by blank and reference surface subtraction (flow cell channel activated by injection of EDC/NHS and inactivated by injection of ethanolamine). The equilibrium dissociation constants (K_{D}) were estimated by global non-linear regression analysis and a reversible 1-step interaction reaching steady state at the end of the analyte injection.

### Metabolic stability analysis

Metabolic stability analysis was performed on a Bruker Avance Ultra High Performance Liquid Chromatography (UHPLC) (Bruker Daltonik, Bremen, Germany) equipped with a CTC-PAL-xt autosampler with cooled sample compartments (10 °C) and a column oven held at 40 °C. The UHPLC eluate was directed to a Bruker Evoq Elite triple quad-ropole MS equipped with a heated electrospray ionization source operated in positive ionization mode. Separation of 15 µL sample was obtained using a Phenomenex (Phenomenex, Torrance, CA, USA) Kinetex XB-C₁₈ column (50 mm × 2.1 mm i.d., 1.7 µm particle size) equipped with a guard column. The analytes were separated using a solvent system consisting of water/acetonitrile (95:5, v/v) (eluent A) and water/acetonitrile (5:95, v/v) (eluent B); both acidified with 0.1% formic acid. The eluent flow rate was maintained at 0.4 mL/min with the following gradient elution profile: 0 min: 0% B; 5 min: 100% B; 6 min: 100% B, followed by three min equilibration at 0% B. Two ion transitions were monitored for all analytes and internal standard.

### Mouse liver microsome assay

The mouse liver microsome assay was performed by transferring 25 µL of a 20 µM solution of the test compound (0.5% DMSO), dissolved in potassium phosphate monobasic buffer (pH 7.4) containing 3 mM MgCl₂, to 9 wells in a microtiter plate. To each well, 25 mL of a 0.5 mg/mL mouse liver (Sigma Aldrich) in buffer was added, and the microtiter plate were pre-incubated for 5 min at 37 °C in a plate shaker at 600 rpm. After pre-incubation, 50 µL 37 °C NADPH regenerating system, consisting of 2 mM NADP+, 10mM glucose-6-phosphate, and 2 U/mL glucose-6-phosphate dehydrogenase dissolved in buffer, was added to start the assay. In triplicates, immediately after adding the NADPH solution, 75 µL was transferred to an Eppendorf tube, containing 75 mL ice-cold acetonitrile with 400 ng/mL internal standard (**7**). Similarly, samples were prepared after 30 min and 60 min incubation at 37 °C. The samples were vortexed for 30 seconds and centrifuged at 16,000 × *g* for 10 minutes before analyzing the supernatant. The individual analyte responses were normalized to the internal standard. Diazepam was used as positive control.

### Mouse plasma assay

The mouse live microsome assay was performed by transferring 75 µL of a 10 µM solution of the test compound (0.5% DMSO), dissolved in PBS buffer (pH 7.4), to 9 wells in a microtiter plate. To each well, 75 µL mouse plasma, harvested from in-house mice into K2-EDTA tubes and centrifuged at 1,600 × g, was added. In triplicates, immediately after adding mouse plasma, 120 µL was transferred to an Eppendorf tube, containing 450 µL ice-cold acetonitrile with 400 ng/mL internal standard (**7**). Similarly, samples were prepared after 60 min and 120 min incubation at 37 °C. The samples were vortexed for 30 seconds and centrifuged at 16,000 × *g* for 10 minutes before analyzing the supernatant. The individual analyte responses were normalized to the internal standard. Enalapril was used as positive control.

### In vivo brain bioavailability

To evaluate the ability of pro-JON-11 (**10**) and JON-11 to penetrate the blood-brain barrier, a brain-to-plasma distribution ratio (B/P ratio) was obtained. All subjects were male C57BL/6JRj mice (24-26 g, Janvier Labs, France) housed in groups of three-four with ad libitum access to food and water. The mice were maintained under diurnal conditions (12-h light/dark cycle with lights on at 7 AM) in a temperature (22±1 °C) and humidity-controlled room (50±10%). Ethical permission for the procedures used in this in vivo study was granted by the Danish Animal Experiments Inspectorate.

Compounds were dissolved in PEG-400 (Sigma) at 20% of the final required volume. The final volume was then made up by 10% (w/v) hydroxypropyl-beta-cyclodextrin (Sigma Aldrich) in sterile saline. The solution was sonicated for 5 min. The JON-11 solution was pH-adjusted to 7.4 with 0.2% 2M NaOH.

Both compounds were injected i.p. at a dose of 10 mg/kg (n=3 for each compound). Two additional mice did not receive any treatment and functioned as 'blank' animals. After 30 min, the animals were sacrificed by decapitation, trunk blood was collected in EDTA-coated tubes and the brain was gently removed. Blood and tissues were temporarily stored on ice until plasma and brain homogenate preparation. Blood plasma was separated from cells by centrifugation for 10 min at 1,500 x *g* at 4 °C and plasma harvested. Brain homogenate was prepared by homogenizing the whole brain with 4 volumes (w:v) of ice-cold sterilised water using a bullet blender (NextAdvance). The plasma and brain homogenate were stored on ice.

Before UHPLC-MS analysis, the plasma and brain samples were prepared by mixing 30 µL of plasma or brain homogenate with 30 µL of blank brain homogenate or blank plasma, respectively. The mixed samples were protein precipitated with 480 µL internal standard solution and centrifuged for 10 min at 15,000 x *g* at 20-25°C. The supernatants were collected and added to HPLC vials for LC-MS analysis, which was performed as described above for metabolic stability analysis.

### Chemical synthesis

Unless otherwise indicated, all reagents used in the examples below are obtained from commercial sources.

The compounds of the general formula I, wherein R₃ is -OH may be prepared as given below from the appropriate substituted pyridazin-3-amine according to the procedures in Examples 1-7 and Examples 28-32.

The compounds of the general formula I, wherein R₃ is different from -OH may be prepared as given below from the appropriate substituted 2-(imidazo[1,2-*b*]pyridazin-2-yl)acetic acid according to the procedures in Examples 8-12 and Examples 33-34.

### Example 1

### Synthesis of 2-(6-(4-Chlorophenyl)imidazo[1,2-b]pyridazin-2-yl)acetic acid (1, JON-11)

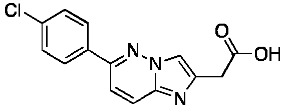

Step 1: 6-(4-chlorophenyl)pyridazin-3-amine (Petrignet et al. 2014) (0.30 g, 1.5 mmol) and ethyl 4-chloroacetoacetate (0.32 mL, 2.2 mmol) were dissolved in EtOH (16 mL) and heated at 80 °C for 48 h. Upon cooling to room temperature, the reaction mixture was evaporated *in vacuo* and the resulting residue was suspended in sat. aq. NaHCO₃ (15 mL) and the mixture was extracted with CH₂Cl₂ (3 × 25 mL). The combined organic phases were washed with water (3 × 25 mL), dried over anhydrous Na₂SO₄, filtered, and evaporated in vacuo. Purification by DCVC (Heptane/EtOAc, 0-100% EtOAc) afforded ethyl 2-(6-(4-chlorophenyl)imidazo[1,2-*b*]pyridazin-2-yl)acetate (**1a**) (0.30 g, 64%) as white sticky solid. ¹H NMR (400 MHz, CD₃OD): δ 8.13 (s, 1H), 8.05 (d, *J* = 8.6 Hz, 2H), 8.01 (d, *J* = 9.5 Hz, 1H), 7.76 (d, *J =* 9.5 Hz, 1H), 7.55 (d, *J =* 8.6 Hz, 2H), 4.21 (q, *J =* 7.1 Hz, 2H), 3.90 (s, 2H), 1.28 (t, *J* = 7.1 Hz, 3H). ¹³C NMR (150 MHz, CD₃OD): δ: 172.1, 152.5, 141.7, 139.5, 137.6, 135.4, 130.4, 129.8, 125.8, 118.3, 117.3, 62.4, 35.7, 14.6.

Step 2: Ethyl 2-(6-(4-chlorophenyl)imidazo[1,2-*b*]pyridazin-2-yl)acetate (140 mg, 0.4 mmol) was dissolved in aq. HCl (4 M, 15 mL) and heated at 45 °C for 48 h. Upon cooling to rt., the reaction mixture was filtered and evaporated in vacuo to afford 2-(6-(4-Chlorophenyl)imidazo[1,2-*b*]pyridazin-2-yl)acetic acid (61 mg, 42%) as light brown solid: mp 195-197 °C. ¹H NMR (400 MHz, CD₃OD): δ 8.48 (d, *J =* 0.7 Hz, 1H), 8.42 (d, *J* = 9.8 Hz, 1H), 8.36 (d, *J =* 9.8 Hz, 1H), 8.18-8.13 (m, 2H), 7.66-7.61 (m, 2H), 4.12 (d, *J =* 0.7 Hz, 2H). ¹³C NMR (150 MHz, CD₃OD): δ 170.3, 156.4, 139.1, 137.1, 133.7, 130.8, 130.4, 130.4, 124.8, 122.9, 119.1, 32.0.

### Example 2

### Synthesis of 2-(6-(3-Chlorophenyl)imidazo[1,2-b]pyridazin-2-yl)acetic acid (2)

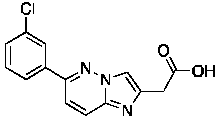

Step 1: Performed a described in example 1 (step 1) using 6-(4-chlorophenyl)pyridazin-3-amine (Guery et al. 2001) (0.25 g, 1.2 mmol) and ethyl 4-chloroacetoacetate (0.3 mL, 1.8 mmol) in EtOH (15 mL) Purification by DCVC (Heptane/EtOAc, 0-100% EtOAc) afforded ethyl 2-(6-(3-chlorophenyl)imidazo[1,2-*b*]pyridazin-2-yl)acetate (99 mg, 26%), as light brown solid: mp 73-77 °C. ¹H NMR (600 MHz, CD₃OD): δ 8.12 (d, *J =* 0.7 Hz, 1H), 8.07-8.05 (m, 1H), 7.98 (d, *J =* 9.5 Hz, 1H), 7.96-7.92 (m, 1H), 7.73 (d, *J =* 9.5 Hz, 1H), 7.52-7.49 (m, 2H), 4.21 (q, *J =* 7.3 Hz, 2H), 3.90 (s, 2H), 1.29 (t, *J* = 7.3 Hz, 3H). ¹³C NMR (150 MHz, CD₃OD): δ 172.1, 152.2, 141.8, 139.5, 138.7, 136.3, 131.8, 131.3, 128.1, 126.6, 125.9, 118.3, 117.4, 62.4, 35.7, 14.6.

Step 2: Performed as describe in example 1 (step 2) using ethyl 2-(6-(3-chlorophenyl)imidazo[1,2-*b*]pyridazin-2-yl)acetate (40 mg, 0.1 mmol) and aq. HCl (4 M, 5 mL) affording 2-(6-(3-chlorophenyl)imidazo[1,2-*b*]pyridazin-2-yl)acetic acid (42 mg, quant.) as brown solid: mp 205-207 °C. ¹H NMR (600 MHz, CD₃OD): δ 8.51 (s, 1H), 8.47-8.44 (m, 1H), 8.41-8.38 (m, 1H), 8.20 (t, *J* = 1.8 Hz, 1H), 8.08 (dt, *J* = 1.5, 7.7 Hz, 1H), 7.66-7.63 (m, 1H), 7.63-7.59 (m, 1H), 4.13 (s, 2H). ¹³C NMR (150 MHz, CD₃OD): δ 171.1, 156.3, 136.9, 136.7, 133.8, 132.7, 132.2, 128.7, 127.3, 125.3, 122.8, 119.2, 31.83.

### Example 3

### Synthesis of 2-(6-(3-Benzyloxy)phenyl)imidazo[1,2-b]pyridazin-2-yl)acetic acid (3)

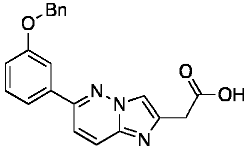

Step 1: Performed a described in example 1 (step 1) using 2-(6-(3-benzyloxy)phenyl) pyridazin-3-amine (Navande et al. 2010) (0.36 g, 1.3 mmol) and ethyl 4-chloroacetoacetate (0.26 mL, 1.9 mmol) in EtOH (15 mL). Purification by DCVC (Heptane/EtOAc, 0-100% EtOAc) affording ethyl 2-(6-(3-(benzyloxy)phenyl)imidazo[1,2-b]pyridazin-2-yl)acetate (0.34 g, 68%) as brown oil. ¹H NMR (600 MHz, CD₃OD): δ 8.12 (s, 1H), 7.98 (d, *J =* 9.5 Hz, 1H), 7.74 (d, *J* = 9.5 Hz, 1H), 7.70-7.68 (m, 1H), 7.63-7.60 (m, 1H), 7.50-7.47 (m, 2H), 7.44 (t, *J* = 7.9 Hz, 1H), 7.41-7.37 (m, 2H), 7.34-7.30 (m, 1H), 7.17-7.14 (m, 1H), 5.19 (s, 2H), 4.21 (q, *J =* 7.1 Hz, 2H), 3.90 (s, 2H), 1.29 (t, *J =* 7.1 Hz, 3H). ¹³C NMR (150 MHz, CD₃OD): δ 172.2, 161.0, 153.5, 141.5, 139.6, 138.7, 138.2, 131.4, 129.7, 129.1, 128.8, 125.7, 120.9, 118.8, 118.1, 117.3, 114.7, 71.4, 62.4, 35.7, 14.6.

Step 2: Performed as describe in example 1 (step 2) using ethyl 2-(6-(3-(benzyloxy)phenyl)imidazo[1,2-b]pyridazin-2-yl)acetate (0.23 g, 0.6 mmol) in THF (6 mL), water (6 mL) and aq. NaOH (0.5 M, 1.2 mL). Evaporation in vacuo afforded 2-(6-(3-benzyloxy)phenyl)imidazo[1,2-*b*]pyridazin-2-yl)acetic acid (0.21 mg, 95%) as brown solid: mp >230 °C. ¹H NMR (600 MHz, CD₃OD): δ 8.04 (s, 1H), 7.92 (d, *J =* 9.5 Hz, 1H), 7.68-7.67 (m, 1H), 7.65 (d, *J =* 9.5 Hz, 1H), 7.60-7.58 (m, 1H), 7.49-7.47 (m, 2H), 7.42 (t, *J* = 7.9 Hz, 1H), 7.40-7.36 (m, 2H), 7.33-7.29 (m, 1H), 7.14-7.11 (m, 1H), 5.18 (s, 2H), 3.74 (s, 2H). ¹³C NMR (150 MHz, CD₃OD): δ 178.2, 160.9, 152.9, 145.5, 139.2, 138.2, 138.4, 131.3, 129.7, 129.1, 128.8, 125.3, 120.9, 117.9, 117.8, 116.8, 114.5, 71.3, 39.7.

### Example 4

### Synthesis of 2-(6-(4-(benzyloxy)phenyl)imidazo[1,2-b]pyridazin-2-yl)acetic acid (4)

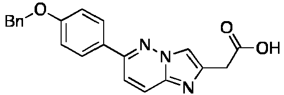

Step 1: Performed a described in example 1 (step 1) using 2-(6-(4-benzyloxy)phenyl) pyridazin-3-amine (Iqbal et al. 2011) (0.51 g, 1.8 mmol) and ethyl 4-chloroacetoacetate (0.38 mL, 2.8 mmol) in EtOH (20 mL). Purification by DCVC (Heptane/EtOAc, 0-100% EtOAc) afforded ethyl 2-(6-(4-benzyloxy)phenyl)imidazo[1,2-b]pyridazin-2-yl)acetate (189 mg, 26%) as light brown solid: mp 126-127 °C. ¹H NMR (600 MHz, CD₃OD): δ 8.06 (d, *J* = 0.7 Hz, 1H), 8.00-7.96 (m, 2H), 7.92 (d, *J* = 9.5 Hz, 1H), 7.69 (d, *J* = 9.5 Hz, 1H), 7.48-7.44 (m, 2H), 7.41-7.36 (m, 2H), 7.34-7.30 (m, 1H), 7.15-7.11 (m, 2H), 5.16 (s, 2H), 4.20 (q, *J =* 7.3 Hz, 2H), 3.87 (s, 2H), 1.28 (t, *J =* 7.2 Hz, 3H). ¹³C NMR (150 MHz, CD₃OD): δ 172.2, 162.2, 153.4, 141.1, 139.4, 138.5, 129.7, 129.7, 129.3, 129.2, 128.8, 125.5, 118.4, 117.1, 116.6, 71.3, 62.4, 35.7, 14.6.

Step 2: Performed as describe in example 1 (step 2) using ethyl 2-(6-(4-(benzyloxy)phenyl)imidazo[1,2-b]pyridazin-2-yl)acetate (62 mg, 0.2 mmol) in aq. HCl (4 M, 8 mL). Upon cooling to rt., the target compound (32 mg, 50%) was isolated by filtration as white solid: mp 193-195 °C. ¹H NMR (600 MHz, DMSO-*d*₆): δ 8.33 (s, 1H), 8.27 (d, *J* = 9.5 Hz, 1H), 8.06 (d, *J* = 9.0 Hz, 2H), 8.03 (d, *J* = 9.5 Hz, 1H), 7.49 (d, *J* = 7.3 Hz, 2H), 7.43-7.40 (m, 2H), 7.37-7.33 (m, 1H), 7.22 (d, *J =* 9.0 Hz, 2H), 5.22 (s, 2H), 3.90 (s, 2H). ¹³C NMR (150 MHz, DMSO-*d*₆): δ 170.7, 160.3, 152.2, 136.7, 135.9, 128.6, 128.5, 127.9, 127.7, 126.7, 123.6, 119.0, 116.1, 115.4, 115.4, 69.4, 33.3.

### Example 5

### Synthesis of 2-(6-(3-Methoxyphenyl)imidazo[1,2-b]pyridazin-2-yl)acetic acid (5)

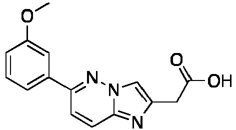

Step 1: Performed a described in example 1 (step 1) using 2-(6-(3-methoxy)phenyl) pyridazin-3-amine (Guery et al. 2001) (0.20 g, 1.0 mmol) and ethyl 4-chloroacetoacetate (0.21 mL, 1.5 mmol) in EtOH (15 mL) Purification by column chromatography (EtOAC/MeOH 15:1) afforded ethyl 2-(6-(3-methoxyphenyl)imidazo[1,2-*b*]pyridazin-2-yl)acetate (100 mg, 32%) as sticky light brown solid. ¹H NMR (400 MHz, CD₃OD): δ 8.10 (s, 1H), 7.96 (d, *J =* 9.5 Hz, 1H), 7.72 (d, *J =* 9.5 Hz, 1H), 7.59-7.55 (m, 2H), 7.46-7.39 (m, 1H), 7.09-7.04 (m, 1H), 4.21 (q, *J* = 7.2 Hz, 2H), 3.89 (s, 2H), 3.88 (s, 3H), 1.28 (t, *J =* 7.2 Hz, 3H). ¹³C NMR (100 MHz, CD₃OD): δ 172.3, 162.1, 153.7, 141.7, 139.8, 138.3, 131.5, 125.8, 120.8, 118.9, 117.5, 117.3, 113.7, 62.6, 56.3, 35.9, 14.8.

Step 2: Performed as describe in example 1 (step 2) using ethyl 2-(6-(3-methoxyphenyl)imidazo[1,2-*b*]pyridazin-2-yl)acetate (94 mg, 0.3 mmol) in aq. HCl (4 M, 10 mL). Upon cooling to rt., the reaction mixture was filtered and evaporated in vacuo to afford 2-(6-(3-Methoxyphenyl)imidazo[1,2-*b*]pyridazin-2-yl)acetic acid (80 mg, 84%) as a brown solid: mp 194-195 °C. ¹H NMR (600 MHz, CD₃OD): δ 8.48 (d, *J* = 0.7 Hz, 1H), 8.44-8.37 (m, 2H), 7.72-7.67 (m, 2H), 7.54-7.49 (m, 1H), 7.19 (ddd, *J =* 0.9, 2.5, 8.3 Hz, 1H), 4.13 (d, *J =* 0.7 Hz, 2H), 3.91 (s, 3H). ¹³C NMR (150 MHz, CD₃OD): δ 171.1, 162.1, 157.5, 136.8, 136.2, 133.4, 131.8, 125.7, 122.4, 121.2, 119.1, 118.5, 114.1, 56.2, 31.8.

### Example 6

### Synthesis of 2-(6-(4-Methoxyphenyl)imidazo[1,2-b]pyridazin-2-yl)acetic acid (6)

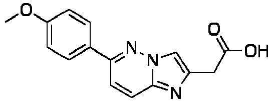

Step 1: Performed a described in example 1 (step 1) using 2-(6-(4-methoxy)phenyl) pyridazin-3-amine **(**Petrignet et al. 2014) (0.26 g, 1.3 mmol) and ethyl 4-chloroacetoacetate (0.28 mL, 2.1 mmol) in EtOH (15 mL). Purification by DCVC (Heptane/EtOAc, 0-100% EtOAc) afforded ethyl 2-(6-(4-methoxyphenyl)imidazo[1,2-*b*]pyridazin-2-yl)acetate (70 mg, 19%) as sticky off-white sticky solid. ¹H NMR (600 MHz, CD₃OD): δ 8.08 (s, 1H), 8.03-7.98 (m, 2H), 7.94 (d, *J* = 9.5 Hz, 1H), 7.72 (d, *J* = 9.5 Hz, 1H), 7.10-7.05 (m, 2H), 4.21 (q, *J* = 7.3 Hz, 2H), 3.89 (s, 2H), 3.88 (s, 3H), 1.28 (t, *J* = 7.2 Hz, 3H). ¹³C NMR (150 MHz, CD₃OD): δ 172.2, 163.2, 153.5, 141.1, 139.4, 129.7, 129.0, 125.5, 118.5, 117.1, 115.6, 62.4, 56.1, 35.7, 14.6

Step 2: Performed as describe in example 1 (step 2) using ethyl 2-(6-(4-methoxyphenyl)imidazo[1,2-*b*]pyridazin-2-yl)acetate (44 mg, 0.2 mmol) dissolved in aq. HCl (4 M, 7 mL). Upon cooling to rt., the reaction mixture filtered and evaporated in vacuo to afford the target compound (43 mg, 87%) as white solid: mp 199-201 °C.¹H NMR (600 MHz, CD₃OD): δ 8.42 (s, 1H), 8.37-8.32 (m, 2H), 8.13-8.10 (m, 2H), 7.16-7.12 (m, 2H), 4.10 (s, 2H), 3.90 (s, 3H). ¹³C NMR (150 MHz, CD₃OD): δ 171.2, 164.3, 157.2, 136.5, 133.2, 130.4, 127.0, 125.1, 122.2, 119.0, 116.0, 56.2, 31.8.

### Example 7

### Synthesis of 2-(6-(3,4-Dichlorophenyl)imidazo[1,2-b]pyridazin-2-yl)acetic acid (7)

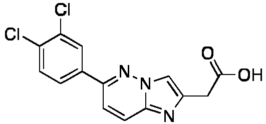

Step 1: 6-Chloropyridazin-3-amine (1.19 g, 9.2 mmol), 3,4-dichlorophenylboronic acid (1.72 g, 9.0 mmol), Pd(PPh₃)₄ (1.04 g, 0.9 mmol), and Na₂CO₃ (1.95 g, 18.4 mmol) were dissolved in EtOH/water 4:1 (90 mL). The resulting reaction mixture was flushed with nitrogen for 5 minutes and heated at reflux under a nitrogen atmosphere for 4 days. The mixture was filtered through a plug celite, diluted with water (100 mL), and extracted with EtOAc (3 × 100 mL). The combined organic phase were dried over anhydrous Na₂SO₄, filtered, and evaporated in vacuo. Purification by column chromatography (CH₂Cl₂/MeOH 25:1) afforded 6-(3,4-dichlorophenyl)pyridazin-3-amine (0.47 mg, 22%) as white solid: mp 165-166 °C. ¹H NMR (400 MHz, CD₃OD): δ 8.11 (d, *J =* 2.2 Hz, 1H), 7.83- 7.79 (m, 2H), 7.61 (d, *J* = 8.9 Hz, 1H), 7.00 (d, *J =* 9.3 Hz, 1H). ¹³C NMR (100 MHz, CD₃OD): δ 161.6, 150.5, 138.7, 134.1, 133.8, 132.1, 128.9, 127.9, 126.7, 117.2.

Step 2: Performed a described in example 1 (step 1) using 6-(3,4-dichlorophenyl)pyridazin-3-amine (0.41 g, 1.2 mmol) and ethyl 4-chloroacetoacetate (3 mL, 2.2 mmol) in EtOH (17mL). Purification by DCVC (Heptane/EtOAc, 0-100% EtOAc) followed by recrystallization from MeCN afforded ethyl 2-(6-(3,4-dichlorophenyl)imidazo[1,2-b]pyridazin-2-yl)acetate (114 mg, 28%) as white solid: mp 157-158 °C. ¹H NMR (600 MHz, DMSO-*d*₆): δ 8.31 (d, *J* = 2.2 Hz, 1H), 8.25 (d, *J* = 0.7 Hz, 1H), 8.17 (dd, *J* = 0.7, 9.5 Hz, 1H), 8.06 (dd, *J* = 2.2, 8.4 Hz, 1H), 7.86 (d, *J =* 9.5 Hz, 1H), 7.82 (d, *J =* 8.4 Hz, 1H), 4.12 (q, *J =* 7.4 Hz, 2H), 3.87 (s, 2H), 1.21 (t, *J =* 7.4 Hz, 3H). ¹³C NMR (150 MHz, CD₃OD): δ 169.8, 148.5, 141.0, 137.2, 135.4, 132.6, 131.8, 131.1, 128.5, 126.8, 125.2, 115.8, 115.4, 60.3, 34.8, 13.9.

Step 3: Performed as describe in example 1 (step 2) using ethyl 2-(6-(3,4-dichlorophenyl)imidazo[1,2-b]pyridazin-2-yl)acetate (82 mg, 0.2 mmol) in aq. HCl (4 M, 9 mL). Upon cooling to rt., 2-(6-(3,4-dichlorophenyl)imidazo[1,2-b]pyridazin-2-yl)acetic acid (41 mg, 50%) was isolated by filtration as white solid: mp 193-195 °C. ¹H NMR

(600 MHz, DMSO-*d*₆): δ 8.35 (d, *J =* 2.2 Hz, 1H), 8.34 (s, 1H), 8.29 (d, *J =* 9.5 Hz, 1H), 8.09 (dd, *J* = 2.2, 8.4 Hz, 1H), 8.03 (d, *J* = 9.5 Hz, 1H), 7.86 (d, *J =* 8.4 Hz, 1H), 3.87 (s, 2H). ¹³C NMR (150 MHz, DMSO-*d*₆): δ 170.6, 150.3, 137.7, 136.1, 134.8, 133.4, 132.1, 131.4, 128.8, 127.2, 124.0, 119.1, 116.4, 33.4.

### Example 8

### Synthesis of Acetoxymethyl 2-(6-(4-chlorophenyl)imidazo[1,2-b]pyridazin-2-yl)acetate (8)

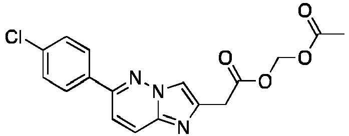

A mixture of 2-(6-(4-Chlorophenyl)imidazo[1,2-b]pyridazin-2-yl)acetic acid (156.3 mg, 0.48 mmol), K₂CO₃ (138.3 mg, 1.0 mmol) and KI (40.9 mg, 0.24 mmol) in DMF (2 mL) was stirred at rt. for 30 minutes. Chloromethyl acetate (64.0 mg, 0.58 mmol) in DMF (1 mL) was added dropwise, and stirred at 70 °C for 2h. Water (15 mL) was added and the aqueous phase was extracted with EtOAc (3×20 mL). The combined organic phasee were dried over anhydrous MgSO₄, filtered, and evaporated *in vacuo* to dryness. Purification by preparative HPLC (gradient 30-100% B over 12 min) afforded the product (93.0 mg, 54%) as white solid. ¹H NMR (600MHz, CD₃OD), δ: 8.15 (s, 1H), 8.07-8.04 (m, 2H), 8.01 (d, *J* = 9.6 Hz, 1H), 7.77 (d, *J =* 9.6 Hz, 1H), 7.56-7.54 (m, 2H), 5.80 (s, 2H), 3.97 (s, 2H), 2.08 (s, 3H). ¹³C NMR (150MHz, CD₃OD), δ: 171.1, 170.5, 152.5, 140.8, 139.4, 137.5, 135.3, 130.3, 129.7, 125.8, 118.3, 117.3, 80.9, 35.2, 20.5.

### Example 9

### Synthesis of (2-(6-(4-Chlorophenyl)imidazo[1,2-b]pyridazin-2-yl)acetoxy)methyl pivalate (9)

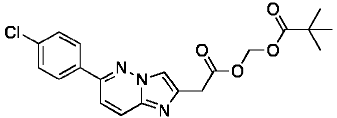

A mixture of 2-(6-(4-Chlorophenyl)imidazo[1,2-*b*]pyridazin-2-yl)acetic acid (81.7 mg, 0.25 mmol), K₂CO₃ (74.3 mg, 0.53 mmol) and KI (29.4 mg, 0.13 mmol) in DMF (1 mL) was stirred at room temperature for 30 minutes. Chloromethyl pivalate (0.05 mL, 0.38 mmol) was added dropwise, and stirred at 50 °C for 3h. Water (5 mL) was added and the aqueous phase was extracted with EtOAc (3×10 mL). The combined organic phases were dried over anhydrous MgSO₄, filtered, and evaporated *in vacuo* to dryness. Purification by column chromatography (EtOAc:Heptane 2:1) afforded the product (70.3 mg, 70%) as off-white solid. ¹H NMR (400MHz, CD₃OD), δ: 8.14 (s, 1H), 8.06-8.03 (m, 2H), 8.01 (d, *J* = 9.6 Hz, 1H), 7.76 (d, *J =* 9.6 Hz, 1H), 7.57-7.53 (m, 2H), 5.81 (s, 2H), 3.96 (s, 2H), 1.16 (s, 9H). ¹³C NMR (100MHz, CD₃OD), δ: 172.4, 170.4, 152.5, 141.5, 140.8, 137.5, 135.3, 130.3, 129.7, 125.7, 118.2, 117.2, 81.1, 38.7, 35.3, 27.2.

### Example 10

### Synthesis of tert.Butyl 2-(6-(4-chlorophenyl)imidazo[1,2-b]pyridazin-2-yl)acetate (10)

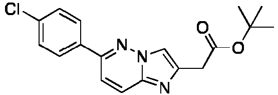

To a solution of 2-(6-(4-Chlorophenyl)imidazo[1,2-*b*]pyridazin-2-yl)acetic acid (51.5 mg, 0.16 mmol, 1.0) in DMF (1 mL), tert.butyl 2,2,2-trichloroacetimidate (0.29 mL, 1.59 mmol) and boron trifluoride diethyl etherate (0.03 mL, 0.24 mmol) were added. The mixture was stirred overnight at 50 °C. A solution of saturated NaHCO₃ was added and the aqueous phase was extracted with EtOAc. The combined organic phases were dried over anhydrous MgSO₄, filtered, and evaporated *in vacuo* to dryness. Purification by column chromatography (EtOAc) afforded the product (22.0 mg, 40%) as white solid. ¹H NMR (400MHz, CD₃OD), δ: 8.11 (s, 1H), 8.07-8.04 (m, 2H), 8.00 (d, *J* = 10.0 Hz, 1H), 7.75 (d, *J =* 9.6 Hz, 1H), 7.57-7.53 (m, 2H), 3.82 (s, 2H), 1.49 (s, 9H). ¹³C NMR (150MHz, CD₃OD), δ: 171.3, 152.4, 142.0, 139.3, 137.4, 135.4, 130.3, 129.7, 125.7, 118.1, 117.1, 82.6, 36.8, 28.3.

### Example 11

### Synthesis of (S)-2-amino-3-(3-(2-(6-(4-chlorophenyl)imidazo[1,2-b]pyridazin-2-yl)acetamido)phenyl)propanoic acid as TFA salt (11)

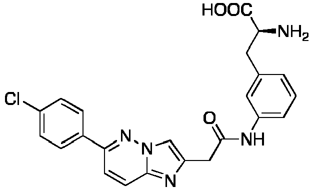

Step1: 2-(6-(4-chlorophenyl)imidazo[1,2-*b*]pyridazin-2-yl)acetic acid hydrochloride (109 mg, 0.3 mmol), N-(3-dimethylaminopropyl)-*N'*-ethylcarbodiimide hydrochloride (78 mg, 0.4 mmol), hydroxybenzotriazole (55 mg, 0.4 mmol), triethylamine (0.14 mL, 1.0 mmol) were dissolved in DMF and stirred for 10 minutes under argon. Methyl (S)-3-(3-aminophenyl)-2-((tert.butoxycarbonyl)amino)propanoate ⁶ (126 mg, 0.4 mmol) in DMF (1.5 mL) was added, and the mixture was stirred overnight at room temperature. A solution of saturated NH₄Cl was added to the mixture, which then, was washed with EtOAc. The combined organic phase was dried over Na₂SO₄, filtered, and evaporated *in vacuo.* Purification by column chromatography (EtOAc/MeOH 50:1) affoprded methyl (*S*)-2-((*tert*-butoxycarbonyl)amino)-3-(3-(2-(6-(4-chlorophenyl)imidazo[1,2-*b*]pyridazin-2-yl)acetamido)phenyl)propanoate (107 mg, 56%) as a pink solid. ¹H NMR (400 MHz, Methanol-*d*₄) δ: 8.16 (s, 1H), 8.07 - 8.03 (m, 2H), 7.92 (d, *J* = 8.9 Hz, 1H), 7.76 (d, *J* = 9.5 Hz, 1H), 7.57 - 7.52 (m, 2H), 7.52 - 7.44 (m, 2H), 7.28 - 7.20 (m, 1H), 6.97 (d, *J* = 7.6 Hz, 1H), 4.37 (d, *J* = 8.8 Hz, 1H), 3.96 (s, 2H), 3.69 (s, 3H), 3.08 (dd, *J* = 13.7, 5.6 Hz, 1H), 2.94 - 2.88 (m, 1H), 1.36 (s, 9H).

Step 2: To a solution of methyl (*S*)-2-((*tert*-butoxycarbonyl)amino)-3-(3-(2-(6-(4-chlorophenyl)imidazo[1,2-*b*]pyridazin-2-yl)acetamido)phenyl)propanoate (87 mg, 0.2 mmol) in MeOH (2 mL) was added with LiOH (12 mg, 0.5 mmol) in water (2 mL), and the mixture was stirred overnight at room temperature. The mixture was evaporated to dryness *in vacuo,* and then, the crude solid was dissolved in DCM (2 mL) and TFA (2 mL). It was stirred for 3h at room temperature before evaporation. Purification by preparative HPLC (gradient 20 - 40% B, eluent A (H₂O/TFA, 100:0.1) and eluent B (MeCN/H₂O/TFA, 90:10:0.1) at a flow rate of 20 mL min⁻¹ , over 8 min) afforded (*S*)-2-amino-3-(3-(2-(6-(4-chlorophenyl)imidazo[1,2-*b*]pyridazin-2-yl)acetamido)phenyl)propanoic acid, TFA salt (45 mg, 44%) as a white solid. ¹H NMR (600 MHz, Methanol-*d*₄) δ: 8.31 (s, 1H), 8.21 (d, *J =* 9.6 Hz, 1H), 8.11 - 8.08 (m, 2H), 8.02 (d, *J =* 9.5 Hz, 1H), 7.67 (d, *J =* 1.9 Hz, 1H), 7.61 - 7.55 (m, 2H), 7.47 - 7.43 (m, 1H), 7.35 (t, *J =* 7.9 Hz, 1H), 7.08 (d, *J =* 7.6 Hz, 1H), 4.24 (dd, *J =* 8.0, 5.3 Hz, 1H), 4.07 (s, 2H), 3.25 - 3.09 (m, 2H). ¹³C NMR (151 MHz, Methanol-*d*₄) δ: 171.2, 169.6, 154.0, 140.2, 139.1, 138.4, 138.1, 136.5, 134.6, 130.7, 130.4, 129.9, 126.6, 124.5, 122.6, 121.0, 120.8, 118.0, 55.1, 37.4, 36.4. HPLC (254 nm): 97%. UPLC-MS: m/z = 448.6 [M-H]⁻, 450.6 [M+H]⁺

### Example 12

### Synthesis of (S)-2-amino-3-(4-(2-(6-(4-chlorophenyl)imidazo[1,2-b]pyridazin-2-yl)acetamido)phenyl)propanoic acid as TFA salt (12)

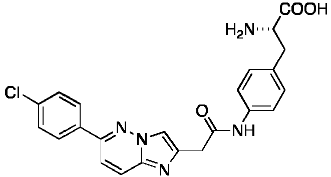

Step1: Performed as described in Example 4 (step 1) using 2-(6-(4-chlorophenyl)imidazo[1,2-*b*]pyridazin-2-yl)acetic acid hydrochloride (95 mg, 0.3 mmol), *N*-(3-dimethylaminopropyl)-*N*'-ethylcarbodiimide hydrochloride (69 mg, 0.4 mmol), hydroxybenzotriazole (48 mg, 0.4 mmol), triethylamine (0.13 mL, 0.9 mmol), methyl (S)-3-(4-aminophenyl)-2-((tert-butoxycarbonyl)amino)propanoate ⁶ (103 mg, 0.4 mmol) in DMF (2 mL). Purification by column chromatography (EtOAc) afforded methyl (S)-2-((*tert*-butoxycarbonyl)amino)-3-(4-(2-(6-(4-chlorophenyl)imidazo[1,2-*b*]pyridazin-2-yl)acetamido)phenyl)propanoate (120 mg, 71%) as a pink solid. ¹H NMR (600 MHz, Methanol-*d*₄) δ: 8.14 (s, 1H), 8.06 - 8.03 (m, 2H), 8.01 (dd, *J* = 9.5, 0.7 Hz, 1H), 7.75 (d, *J* = 9.6 Hz, 1H), 7.57 - 7.50 (m, 4H), 7.17 (d, *J* = 8.5 Hz, 2H), 4.34 (dd, *J =* 8.9, 5.6 Hz, 1H), 3.95 (s, 2H), 3.69 (s, 3H), 3.07 (dd, *J =* 13.9, 5.6 Hz, 1H), 2.91 - 2.87 (m, 1H), 1.38 (s, 9H).

Step 2: Performed as described in Example 4 (step 2) using methyl (*S*)-2-((*tert-*butoxycarbonyl)amino)-3-(3-(4-(6-(4-chlorophenyl)imidazo[1,2-b]pyridazin-2-yl)acetamido)-phenyl)propanoate (97 mg, 0.2 mmol), LiOH (10 mg, 0.3 mmol), MeOH (2 mL), water (2 mL), DCM (3 mL) and TFA (3 mL). Purification by preparative HPLC (gradient 10 - 100% B, eluent A (H₂O/TFA, 100:0.1) and eluent B (MeCN/H₂O/TFA, 90:10:0.1) at a flow rate of 20 mL min⁻¹, over 8 min) afforded (*S*)-2-amino-3-(4-(2-(6-(4-chlorophenyl)imidazo[1,2-*b*]pyridazin-2-yl)acetamido)-phenyl)propanoic acid TFA salt (89 mg, 77%) as a white solid. ¹H NMR (600 MHz, Methanol-*d*₄) δ: 8.38 (s, 1H), 8.29 (d, *J* = 9.6 Hz, 1H), 8.15 (d, *J =* 9.6 Hz, 1H), 8.13 - 8.10 (m, 2H), 7.61 (tt, *J =* 9.4, 2.3 Hz, 4H), 7.29 - 7.26 (m, 2H), 4.23 (dd, *J* = 7.7, 5.5 Hz, 1H), 4.12 (s, 2H), 3.30 - 3.27 (m, 1H), 3.13 (dd, *J* = 14.6, 7.7 Hz, 1H). ¹³C NMR (151 MHz, Methanol-*d*₄) δ 171.2, 168.6, 154.9, 139.3, 138.4, 137.8, 137.1, 134.2, 131.6, 131.0, 130.5, 130.0, 123.7, 122.4, 121.9, 118.4, 55.1, 36.8, 35.4. HPLC (254 nm): 95%. UPLC-MS: m/z = 448.6 [M-H]⁻, 450.6 [M+H]⁺

### Example 13

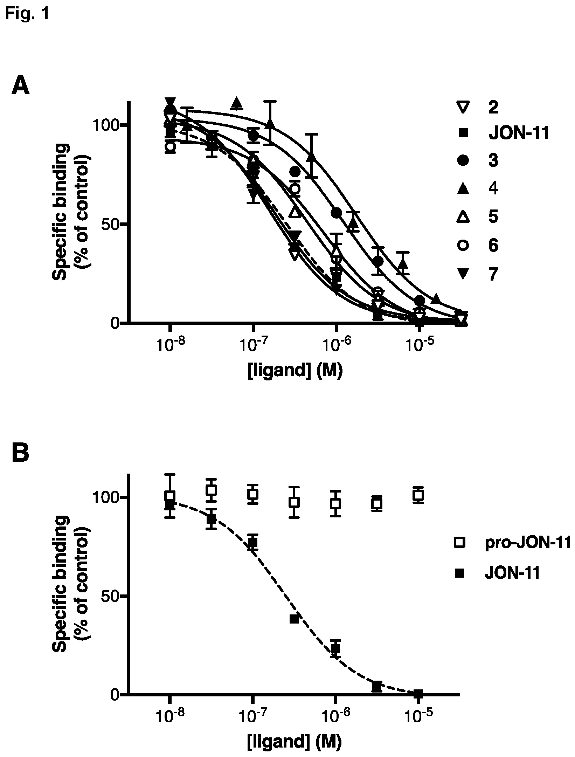

### JON-11 and JON-11 analogues bind with high affinity to specific GHB sites in rat brain synaptic membranes (fig. 1)

JON-11, related analogues, and pro-JON-11(**10**) were tested in the [³H]NCS-382 binding assay (Wellendorph et al., 2005). A) JON-11 (*K*ᵢ value of 0.22 µM) and the analogues **2-7** (respective *K*ₗ values of 0.23, 0.51, 2.2, 0.44, 0.83 and 0.19 µM) all inhibited binding in a concentration-dependent manner. B) The proform of JON-11, pro-JON-11 (**10**), failed to displace radioligand binding at concentrations up to 10 µM and thus only binds to the binding site after being cleaved into the acid.

### Example 14

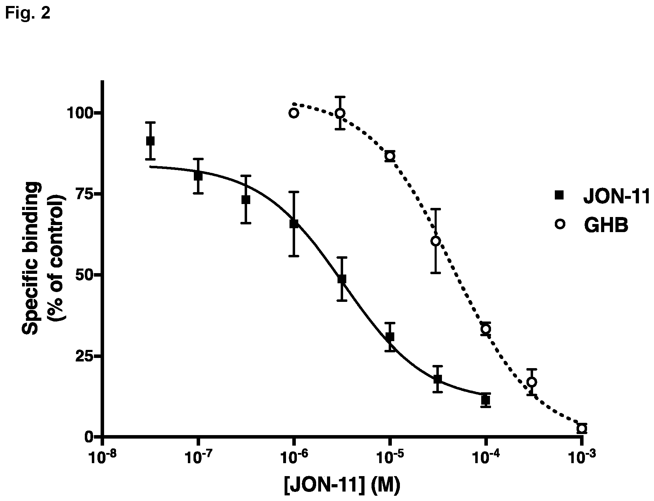

### JON-11 binds with higher affinity than GHB to recombinant CaMK2a expressed in HEK cells (fig. 2)

Human/rat CaMK2a expressed in HEK cells was assayed in an in-house established [³H]HOCPCA filtration binding assay performed on whole cell lysates of CaMK2a-transfected HEK293T cells. JON-11 was able to concentration-dependently inhibit radioligand binding (*K*ᵢ value 3.5 µM), displaying 14x higher affinity compared to GHB itself (*K*ᵢ value of 50.4 µM).

### Example 15

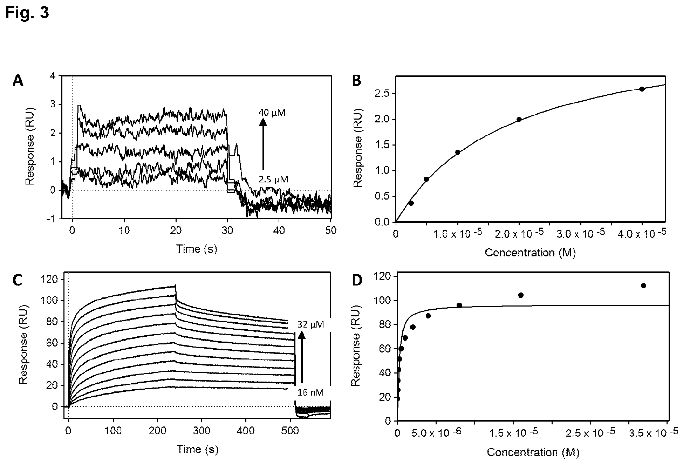

### JON-11 displays SPR binding to recombinant CaMK2a immobilized on a biosensor chip (fig. 3)

The SPR experiments confirmed an interaction between A) the lower molecular weight compound JON-11 and CaMK2a although at low response level. B) From steady state affinity analysis the K_{D} was estimated to 20 µM. C) As a positive control, calmodulin was similarly injected in a buffer supplemented with Ca²⁺ (500 µM) over immobilized CaMK2a, which revealed a strong complex formation with very slow dissociation rate. The interaction had to be actively broken using a regeneration solution (100 µM EDTA) so that the signal returned to baseline before new injection of calmodulin. D) From steady state affinity analysis the K_{D} was estimated to 192 nM.

### Example 16

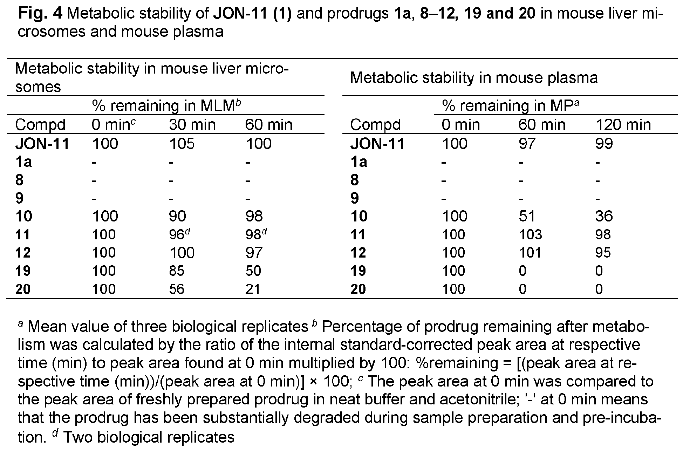

### JON-11 and pro-JON-11 display high stability in plasma and liver microsomes (fig. 4)

The metabolic stabilities of JON-11 and the prodrugs **1a**, **8-12, 19** and **20** were determined *in vitro* in rat plasma and rat liver microsomes. The remaining percentages of the compounds after incubation are summarized. The tert.butyl ester (**10**) showed high stability in rat liver microsomes and fair stability in rat plasma (t_{1/2} ≈ 60 min), however, prodrugs **1a, 8** and **9** were highly chemical/metabolic unstable and converted to the desirable parent compound JON-11 instantly. Chemical or enzymatic degradation of JON-11 was not observed during the metabolic stability study, correlating well with the high bioavailability observed in the brain exposure study. The corresponding loss of prodrug compounds and formation of parent drug JON-11 was determined by UHPLC-MS.

### Example 17

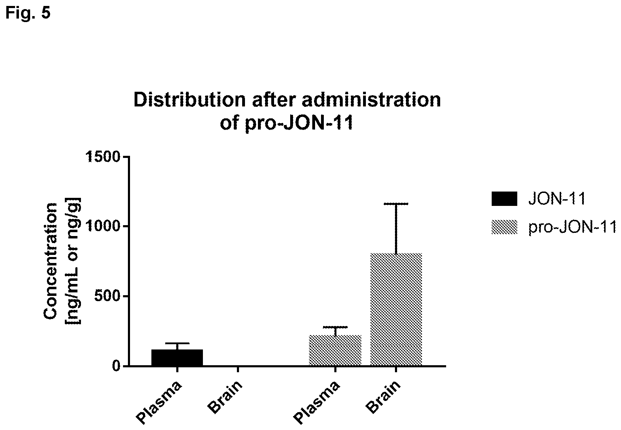

### pro-JON-11 enters the brain in vivo in mice (fig. 5)

Plasma and brain exposure of JON-11 and pro-JON-11 were evaluated in male mice. It was found that JON-11 exhibits seemingly very low brain passage (B/P ratio 0.05 in mice, dose 10 mg/kg i.p. injected). However, the prodrug, pro-JON-11 enters the brain (B/P ratio 2.24, dose 10 mg/kg i.p.).

### Future examples

### Example 18

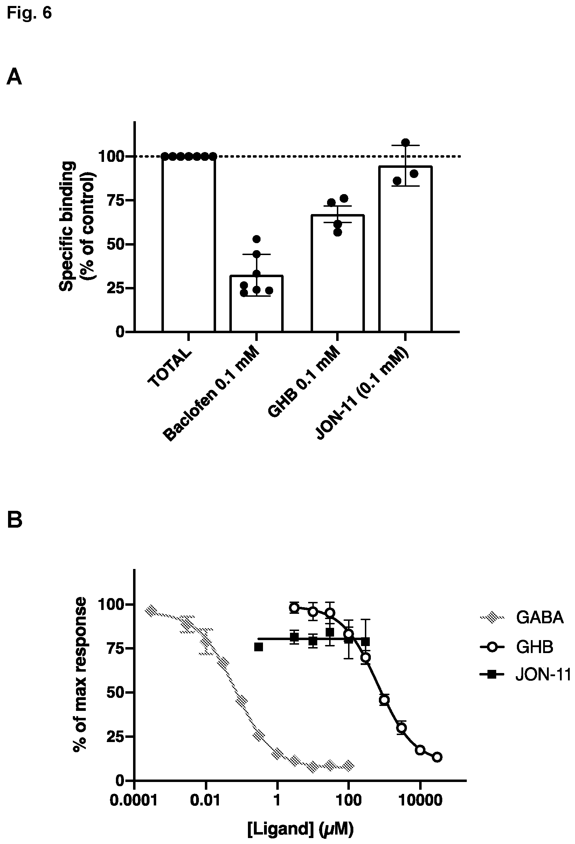

### GABA_{B} receptor pharmacological assays (fig. 6)

To assess affinity of JON-11 for the GABA_{B} receptor, we employed a GABA_{B} binding assay using rat brain synaptic membranes (Wellendorph et al., 2005). A) JON-11 showed no ability to compete with [³H]GABA at 100 µM (cf. baclofen and GHB). B) JON-11 was further assessed for functional activity using CHO cells stably expressing GABA_{B(1b,2)} receptors (Rajalu et al., 2015). As shown, JON-11 is not a GABA_{B} agonist.

### Example 19

### CaMK2a pT286 autophosphorylation assay

We will assess functional activity of selected analogues in intact CaMK2a-transfected HEK293T cells using the well-described pT286 assay (Kool et al., 2016). To this end, HEK293T cells will be transfected with CaMK2a and at 48 hrs post-transfection, different concentrations compounds will be bath-applied to the cells alone or together with Ca²⁺. After different incubation times, cells will be lysed using 1x RIPA buffer supplemented with phosphatase and protease inhibitors (Phosphatase inhibitor cocktail 3 #P0044 (Sigma), Phosphatase inhibitor cocktail 2 #P5726 (Sigma) and complete EDTA protease inhibitors (Roche)). Autophosphorylation will be assessed by Western blot analysis, comparing the total level of CaMK2a (quantified using anti-myc-Alexa488, MA1980-A488, ThermoFisher Scientific) to the level of phosphorylated CaMK2a (pThr286: #12716S, NewEngland BioLabs; pThr306: #NBP2-60767, Novus Biologicals; goat anti-rabbit HRP: #PI-1000 X0126, Vector). Levels of pT286 CaMK2a will be normalized to total CaMK2a to detect changes in autophosphorylation. Cells only treated with vehicle, ionomycin or calcium chelator will be used as controls.

### Example 20

### Evaluation of pro-JON-11 stability and cleavage to JON-11 in brain homogenate

To assess stability of pro-JON-11 and rate of formation of JON-11 following brain delivery of pro-JON-11, we will determined *in vitro* stability in mouse brain homogenate following the procedure described for stability test in mouse plasma and liver microsomes. The loss of prodrug compound and formation of parent drug JON-11 will be determined by UHPLC-MS.

### Example 21

### Synthesis of LAT1 prodrugs of JON-11 and analogues

To probe the utility of the BBB L-type amino acid transporter 1 (LAT1, SLC7A5) for drug delivery, we will synthesize various JON-11 derivatives conjugated with selected amino acid residues. This will be done following the procedure describe in examples 11 and 12.

### Example 22

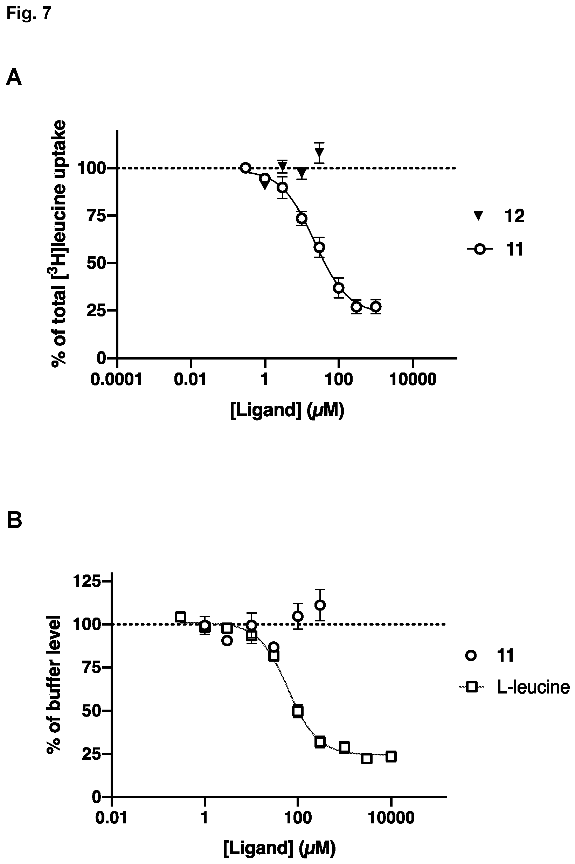

### Evaluation of LAT-1 prodrugs for drug delivery (fig. 7)

To test selected prodrugs for substrate activity at LAT-1, we characterised the uptake and efflux of [³H]leucine in the presence of compounds. For this purpose we used HEK293T cells endogenously expressing a functionally competent LAT-1, and performed assays according to Chien et al., 2018 to measure A) uptake and B) relative exchange efflux rate (L-leucine as control). As shown, JON-11 prodrug **11** is a LAT1 inhibitor.

### Example 23

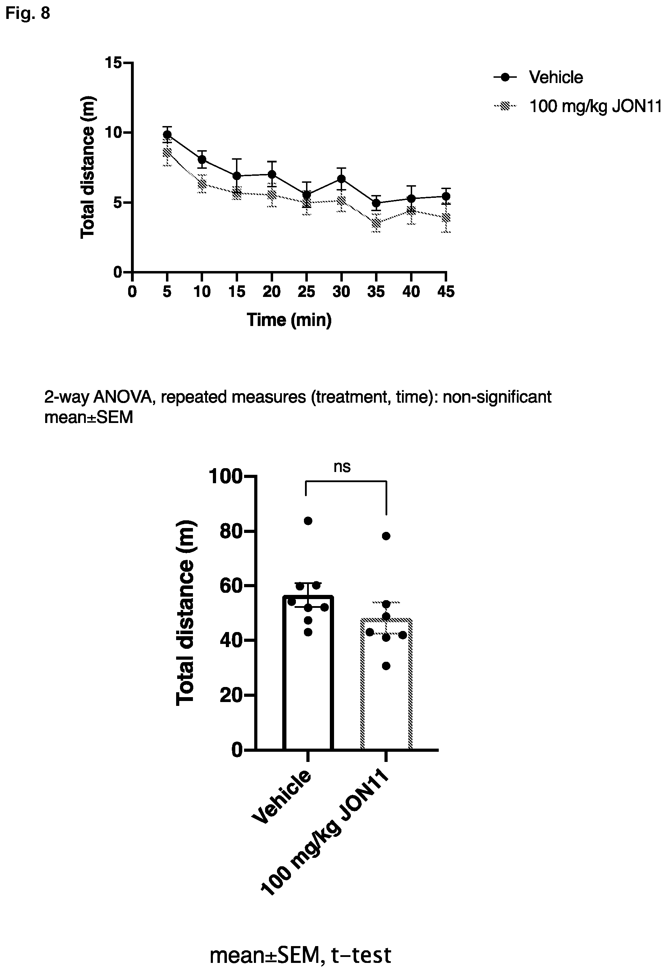

### Evaluation of locomotor activity of selected compounds in mice (fig. 8)

To determine locomotor effects (e.g. sedation or hyperactivity) compounds were assessed after systemic administration to mice. Mice (typically n=5-8) were administered selected compounds, e.g. JON-11 and pro-JON-11 and vehicle controls, and placed in transparent cages (L: 37 cm x W: 21 cm x H: 15 cm). Locomotor activity were measured via a camera mounted above the arena. Mice will be recorded for 45 min and data collected in 5-min intervals. Data will be stored on a computer equipped with Ethovision XT (Noldus). Figure 8 shows that JON-11 does not affect locomotor activity.

### Example 24

### Testing selected compounds for neuroprotective effects in a mouse model of focal ischemia

Using the photothrombotic model for focal ischemia we will test selected compounds, e.g. JON-11 and pro-JON-11 for neuroprotective effects (compared to vehicle and sham animals, all injected i.p.). To this end, a photochemical lesion will be introduced to male C57BL/6J mice (8-10 weeks) weighing ~ 23-30 g as previously described (Clarkson et al., 2010). Under anesthesia with isoflurane (2% to 2.5% in O₂) mice will be placed in a stereotactic apparatus, the skull exposed through a midline incision, cleared of connective tissue and dried. A cold light source attached to a 40x objective providing a 2-mm diameter illumination will be positioned 1.5 mm lateral from bregma. Then, 0.2 ml of Rose Bengal (Sigma-Aldrich, 10 mg/ml in normal saline) is administered i.p. After 5 min, the brain is illuminated through the exposed intact skull for 15 min, while keeping body temperature at 37.0 ± 0.3 °C degrees using a heating pad (Harvard apparatus, Holliston, MA, USA). 3 or 7 days later resulting infarct sizes will be measured using histochemistry. Sectioned brains fixed with 4% PFA are cut in µm sections free-floating in anti-freeze media. Sections are mounted, stained for cresyl violet and the infarct volumes determined by measuring every 6th section through the entire infarct as described and reported as infarct volumes (Clarkson et al., 2010). Analyses will be performed by an observer blinded to the treatment groups.

### Example 25

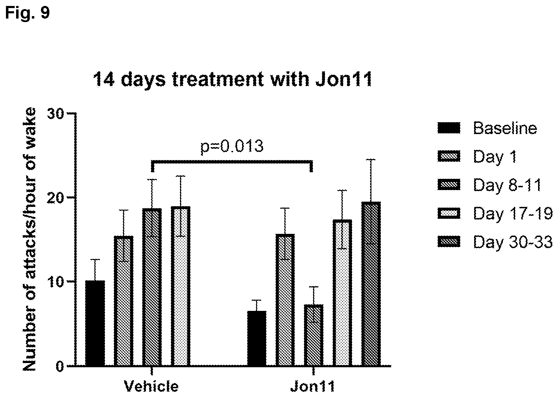

### Evaluation of selected compounds in the DTA mouse model of narcolepsy (fig.9)

Using the DTA mouse model (Tabuchi et al., 2014) we determined changes in sleep-wake EEG/EMG patterns (including cataplexy) at different time points (1 day and 8-11 days) under the influence of JON-11. After drug cessation day 14, EEG/EMG changes were mapped up to day 33. Under anesthesia with isoflurane (2% to 2.5% in O₂) electrodes were placed in the scull and neck muscles of the mice. After 5-10 days recovery the electrodes are connected to a recording system, and EEG/EMG signals are recorded with synchronised video recordings. From the data, sleep/wake parameters and cataplexy episodes are scored and calculated. Analyses were performed by an observer blinded to the treatment groups.

### Example 26

### Evaluation of selected compounds in a hypocretin knock-out mouse model of narcolepsy

Using the hypocretin knock-out mouse model we will determine changes in sleep-wake EEG/EMG patterns (including cataplexy) at different time points (1 day to 3 weeks) under the influence of selected compounds, e.g. JON-11 and pro-JON-11. After drug cessation, EEG/EMG changes will be further mapped for up to 4 weeks. Under anesthesia with isoflurane (2% to 2.5% in O₂) electrodes will be placed in the scull and neck muscles of the mice. After 5-10 days recovery the electrodes are connected to a recording system, and EEG/EMG signals are recorded with synchronised video recordings. From the data, sleep/wake parameters and cataplexy episodes are scored and calculated. Analyses will be performed by an observer blinded to the treatment groups.

### Example 27

### Synthesis of sodium 2-(6-(4-Chlorophenyl)imidazo[1,2-b]pyridazin-2-yl)acetic acetate (sodium salt of 1 from example 1) (13)

The sodium salt of 1 was prepared by dissolving **1** (216 mg, 0.7 mmol) in ethanol (2 ml) and NaOH (aq) (1.5 mL, 0.7 mmol, 0.5M Tritisol) was added. The solvent was removed in vacuo to give the product (215 mg) as white solid.

### Example 28

### Synthesis of 2-(6-(4-nitrophenyl)imidazo[1,2-b]pyridazin-2-yl)acetic acid (14)

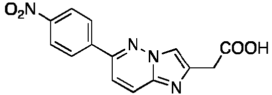

Step1: 6-(4-nitrophenyl)pyridazine-3-amine (270 mg, 1.2 mmol) and ethyl 4-chloroacetoacetate (0.26 mL, 1.9 mmol) were dissolved in 20 mL of ethanol. The reaction mixture was stirred at reflux for 48 hours. After evaporating the solvent, aqueous sat NaHCO₃ was added. The mixture was extracted with DCM. The combined organic phases were dried over Na₂SO₄, filtered, and evaporated in vacuo. Purification by column chromatography (EtOAc/Heptane 8:2) furnished ethyl 2-(6-(4-nitrophenyl)imidazo[1,2-*b*]pyridazin-2-yl)acetate (199.3 mg, 49%). ¹H NMR (400 MHz, DMSO-*d*₆) δ 8.44 - 8.32 (m, 4H), 8.30 (s, 1H), 8.23 (d, *J* = 9.6 Hz, 1H), 7.91 (d, *J* = 9.6 Hz, 1H), 4.13 (q, *J =* 7.1 Hz, 2H), 3.89 (s, 2H), 1.21 (t, *J =* 7.1 Hz, 3H).

Step 2: Ethyl 2-(6-(4-nitrophenyl)imidazo[1,2-b]pyridazin-2-yl)acetate (53.1 mg, 0.16 mmol) was dissolved in 4M HCl (20 mL) and stirred overnight at 50°C. After extraction with ethyl acetate, the aqueous phase was lyophilized to furnish 2-(6-(4-nitrophenyl)imidazo[1,2-*b*]pyridazin-2-yl)acetic acid, hydrochloride (18,2 mg, 37 %) as a pale yellow solid: m.p 190-192°C. ¹H NMR (400 MHz, Methanol-*d*₄) δ 8.59 - 8.38 (m, 7H), 4.15 (s, 2H).¹³C NMR (151 MHz, Methanol-*d*₄) δ 170.91, 155.65, 151.10, 140.55, 136.80, 133.97, 130.10, 125.38, 122.88, 119.16, 31.66. HPLC(254nm)=98%.

### Example 29

### Synthesis of 2-(6-(4-aminophenyl)imidazo[1,2-b]pyridazin-2-yl)acetic acid (15)

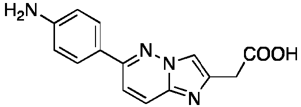

Step1: Ethyl 2-(6-(4-nitrophenyl)imidazo[1,2-b]pyridazin-2-yl)acetate (165 mg, 0.5 mmol) and SnCl₂ (480 mg, 2.5 mmol) were dissolved in 30 mL of methanol and stirred overnight under reflux. After reducing the solvent in vacuo and adding 10 mL of EtOAc, the mixture was alkalized to pH=7 using 5N sodium hydroxide. After filtration through a short pad of celite and evaporation, ethyl 2-(6-(4-aminophenyl)imidazo[1,2-b]pyridazin-2-yl)acetate (70.3 mg, 49%) was afforded. ¹H NMR (400 MHz, DMSO-*d*₆) δ 8.08 (s, 1H), 7.97 (d, *J =* 9.6 Hz, 1H), 7.76 (d, *J =* 8.6 Hz, 2H), 7.64 (d, *J* = 9.5 Hz, 1H), 6.67 (d, *J* = 8.7 Hz, 2H), 5.62 (s, 2H), 3.83 (s, 2H), 3.64 (s, 3H).

Step2: Performed as described in example 1 (step 2) using ethyl 2-(6-(4-aminophenyl)imidazo[1,2-b]pyridazin-2-yl)acetate (68 mg, 0.24 mmol) and 4M HCl (20 mL). Lyophilization of the aqueous phase furnished 2-(6-(4-aminophenyl)imidazo[1,2-*b*]pyridazin-2-yl)acetic acid hydrochloride (52,8 mg, 84 %) as an yellow solid: m.p 241-243°C. ¹H NMR (400 MHz, Methanol-*d*₄) δ 8.48 (dd, *J =* 24.6, 10.3 Hz, 3H), 8.34 (d, *J =* 8.6 Hz, 2H), 7.65 (d, *J* = 8.5 Hz, 2H), 4.15 (s, 2H). ¹³C NMR (151 MHz, Methanol-*d*₄) δ 129.27, 123.92, 123.66, 121.30 (d, *J =* 8.8 Hz), 117.66, 30.19. HPLC(254nm)=100%.

### Example 30

### Synthesis of 2-(6-(4-hydroxyphenyl)imidazo[1,2-b]pyridazin-2-yl)acetic acid (16)

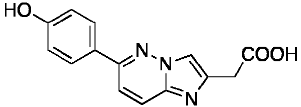

Step1: Performed as described in example 1-1 (step 1) using 4-(6-aminopyridazin-3-yl)phenol (300 mg, 1.6 mmol), ethyl 4-chloroacetoacetate (0.3 mL, 2.2 mmol) and ethanol (20 mL). Purification by column chromatography (EtOAc/Heptane 9:1) furnished ethyl 2-(6-(4-hydroxyphenyl)imidazo[1,2-*b*]pyridazin-2-yl)acetate (199.3 mg, 49%). ¹H NMR (400 MHz, DMSO-*d*₆) δ 9.94 (s, 1H), 8.13 (s, 1H), 8.05 (d, *J* = 9.6 Hz, 1H), 7.90 (d, *J =* 8.7 Hz, 2H), 7.69 (d, *J =* 9.6 Hz, 1H), 6.92 (d, *J* = 8.7 Hz, 2H), 4.12 (q, *J* = 7.1 Hz, 2H), 3.83 (s, 2H), 1.21 (t, *J =* 7.1 Hz, 3H).

Step2: Performed as described in example 1 (step 2) using ethyl 2-(6-(4-hydroxyphenyl)imidazo[1,2-*b*]pyridazin-2-yl)acetate (165.6 mg, 0.56 mmol) and 4M HCl (20 mL). Lyophilization of the aqueous phase furnished 2-(6-(4-hydroxyphenyl)imidazo[1,2-b]pyridazin-2-yl)acetic acid hydrochloride (119.4 mg, 80 %) as a white solid: m.p 218-220°C.: ¹H NMR (400 MHz, Methanol-*d*₄) δ 8.41 (s, 1H), 8.33 (d, *J =* 1.2 Hz, 2H), 8.03 (d, *J* = 8.8 Hz, 2H), 6.98 (d, *J =* 8.8 Hz, 2H), 4.11 (s, 2H).¹³C NMR (151 MHz, Methanol-*d*₄) δ 162.44 (d, *J* = 3.8 Hz), 157.45 (d, *J* = 7.3 Hz), 136.25 (d, *J* = 12.0 Hz), 132.69, 130.41 (d, *J =* 1.8 Hz), 125.46 (d, *J =* 4.9 Hz), 125.15, 121.80 (d, *J =* 6.7 Hz), 118.82, 117.26, 31.53. HPLC(254nm)=97%.

### Example 31

### Synthesis of 2-(6-(pyridin-4-yl)imidazo[1,2-b]pyridazin-2-yl)acetic acid (17)

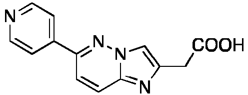

Step1: To a thick-wall borosilicate glass vial were added 3-amino-6-chloropyridazine (150 mg, 1.2 mmol), K₂CO₃ (240 mg, 1.7 mmol), 4-pyridinylboronic acid (171 mg, 1.4 mmol), Pd(PPh₃)₄ (67 mg, 6% mmol) in ethanol-water (4:1, 2 mL). The mixture was degassed with N₂ for 10 minutes, which then was irradiated in a microwave reactor at 120 °C for 50 min. The reaction mixture was concentrated under reduced pressure. Purification by column chromatography (EtOAc/MeOH 9:2) furnished 6-(pyridin-4-yl)pyridazin-3-amine (93 mg, 47%).

Step2: Performed as described in example 1 (step 1) using 6-(pyridin-4-yl)pyridazin-3-amine (66 mg, 0.4 mmol), ethyl 4-chloroacetoacetate (78.0 µL, 0.6 mmol) and ethanol (4 mL). Purification by column chromatography (EtOAc/MeOH 9:1) furnished ethyl 2-(6-(pyridin-4-yl)imidazo[1,2-*b*]pyridazin-2-yl)acetate (33 mg, 30%).

Step3: Performed as described in example 1 (step 2) using ethyl 2-(6-(pyridin-4-yl)imidazo[1,2-b]pyridazin-2-yl)acetate (33 mg, 0.1 mmol) and 4M HCl (7 mL). Lyophilization of the aqueous phase furnished Synthesis of 2-(6-(pyridin-4-yl)imidazo[1,2-b]pyridazin-2-yl)acetic acid hydrochloride (23 mg, 77 %). HPLC(254nm)=87%.

### Example 32

### Synthesis of 2-(6-(2-chloropyridin-4-yl)imidazo[1,2-b]pyridazin-2-yl)acetic acid (18)

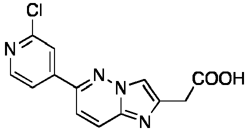

Step1: Performed as described in example 5 (step 1) using 3-amino-6-bromopyridazine (193.2 mg, 1.2 mmol), K₂CO₃ (218.4 mg, 1.9 mmol), 2-chloro-4-pyridinylboronic acid (199.0 mg, 1.5 mmol), Pd(PPh₃)₄ (73.0 mg, 7% mmol) in ethanol-water (4:1, 20 mL) at reflux for overnight. Purification by column chromatography (EtOAc) furnished 6-(2-chloropyridin-4-yl)pyridazin-3-amine (145.6 mg, 73%) as a yellow solid. ¹H NMR (400 MHz, DMSO-*d*₆) δ 8.47 (d, *J* = 5.2 Hz, 1H), 8.04 (s, 1H), 7.99 (d, *J* = 9.4 Hz, 2H), 6.87 (d, *J* = 7.3 Hz, 1H), 6.86 (s, 2H).

### Step2:

Performed as described in example 1 (step 1) using 6-(2-chloropyridin-4-yl)pyridazin-3-amine (144 mg, 0.7 mmol), ethyl 4-chloroacetoacetate (0.15 mL, 1.1 mmol) and ethanol (20 mL). Purification by column chromatography (EtOAc/MeOH 9:1) furnished ethyl 2-(6-(2-chloropyridin-4-yl)imidazo[1,2-b]pyridazin-2-yl)acetate (20.8 mg, 9%). ¹H NMR (400 MHz, DMSO-*d*₆) δ 8.61 (d, *J =* 5.2 Hz, 1H), 8.32 (s, 1H), 8.25 (d, *J =* 9.6 Hz, 1H), 8.17 (d, *J =* 1.5 Hz, 1H), 8.08 (dd, *J* = 5.2, 1.6 Hz, 1H), 7.95 (d, *J* = 9.5 Hz, 1H), 4.13 (q, *J =* 7.1 Hz, 2H), 3.89 (s, 2H), 1.21 (t, *J* = 7.1 Hz, 3H).

Step3: Performed as described in example 1-1 (step 2) using ethyl 2-(6-(2-chloropyridin-4-yl)imidazo[1,2-b]pyridazin-2-yl)acetate (20 mg, 0.06 mmol) and 4M HCl (20 mL). Lyophilization of the aqueous phase furnished 2-(6-(2-chloropyridin-4-yl)imidazo[1,2-b]pyridazin-2-yl)acetic acid hydrochloride (15.5 mg, 85 %) as an off-white solid.: m.p 192-194°C. ¹H NMR (400 MHz, Methanol-*d*₄) δ 8.66 - 8.53 (m, 3H), 8.48 (d, *J =* 9.7 Hz, 1H), 8.24 (d, *J =* 1.5 Hz, 1H), 8.11 (dd, *J* = 5.3, 1.6 Hz, 1H), 4.16 (s, 2H).¹³C NMR (151 MHz, Methanol-*d*₄) δ 170.82, 169.89, 154.16 (d, *J* = 5.8 Hz), 153.75, 152.02, 145.76 (d, *J =* 3.3 Hz), 137.16, 137.06, 134.19, 133.65, 125.16, 125.08, 123.56, 123.23, 123.15, 121.87, 119.36, 119.25, 35.38, 31.66, 31.58. HPLC(254nm)=99%.

### Example 33

### Synthesis of neopentyl 2-(6-(4-chlorophenyl)imidazo[1,2-b]pyridazin-2-yl)acetate (19)

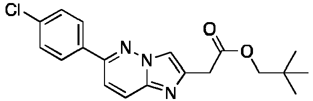

To a solution of 1 (JON-11 (Krall et al)) (87.3 mg, 0.3 mmol), 4-dimethylaminopyridine (7.3 mg, 5.4% mmol) and 2,2-dimethylpropan-1-ol (35.5 mg, 0.4 mmol) in DMF (2 mL) at 0 °C was added *N*-(3-dimethylaminopropyl)-*N'*-ethylcarbodiimide hydrochloride (103.8 mg, 0.5 mmol) dissolved in DMF (1 mL). The mixture was then allowed to room temperature and stirred for overnight. A solution of saturated NH₄Cl was added to the mixture, which then, was extracted with EtOAc. The combined organic phases were washed by brine, dried over Na₂SO₄, filtered, and evaporated *in vacuo.* Purification by column chromatography (EtOAc/Heptane 5:1) furnished neopentyl 2-(6-(4-chlorophenyl)imidazo[1,2-b]pyridazin-2-yl)acetate (45.6 mg, 47%) as a white solid. ¹H NMR (600 MHz, Methanol-*d*₄) δ 8.14 (s, 1H), 8.07 - 8.04 (m, 2H), 8.01 (d, *J* = 9.5 Hz, 1H), 7.76 (d, *J* = 9.5 Hz, 1H), 7.56 - 7.53 (m, 2H), 3.94 (s, 2H), 3.86 (s, 2H), 0.93 (s, 9H). ¹³C NMR (151 MHz, Methanol-*d*₄) δ 172.0, 152.5, 141.7, 139.4, 137.5, 135.3, 130.3, 129.7, 125.7, 118.2, 117.2, 75.4, 35.5, 32.2, 26.7. HPLC (254 nm): 100%.

### Example 34

### Synthesis of isopropyl 2-(6-(4-chlorophenyl)imidazo[1,2-b]pyridazin-2-yl)acetate (20)

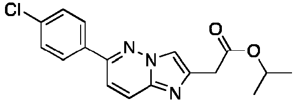

Performed as described in example 7 using JON-11 (Krall et. al) (87.9 mg, 0.3 mmol), 4-dimethylaminopyridine (9.2 mg, 5.4% mmol), isopropanol (0.04 mL, 0.5 mmol), *N*-(3-dimethylaminopropyl)-*N'*-ethylcarbodiimide hydrochloride (104.7 mg, 0.5 mmol) and DMF (3 mL). Purification by column chromatography (EtOAc/Heptane 5:1) furnished isopropyl 2-(6-(4-chlorophenyl)imidazo[1,2-b]pyridazin-2-yl)acetate (53.1 mg, 60%) as an off-white solid. ¹H NMR (600 MHz, Methanol-*d*₄) δ 8.12 (s, 1H), 8.07 - 8.03 (m, 2H), 8.00 (dd, *J* = 9.5, 0.7 Hz, 1H), 7.75 (d, *J* = 9.5 Hz, 1H), 7.57 - 7.53 (m, 2H), 5.05 (hept, *J =* 6.3 Hz, 1H), 3.87 (s, 2H), 1.28 (s, 3H), 1.27 (s, 3H). ¹³C NMR (151 MHz, Methanol-*d*₄) δ 171.5, 152.4, 141.7, 139.3, 137.4, 135.3, 130.3, 129.7, 125.7, 118.2, 117.2, 70.0, 35.9, 22.0. HPLC (254 nm): 100%.

### References

Bay, T.; Eghorn, L. F.; Klein, A. B.; Wellendorph, P. GHB receptor targets in the CNS: focus on high-affinity binding sites. Biochem. Pharmacol., 2014, 87, 220-8.
Chien, H.C., Colas, C., Finke, K., Springer, S., Stoner, L., Zur, A.A., Venteicher, B., Campbell, J., Hall, C., Flint, A., Augustyn, E., Hernandez, C., Heeren, N., Hansen, L., Anthony, A., Bauer, J., Fotiadis, D., Schlessinger A., Giacomini, K.M., Thomas, A.A. Reevaluating the Substrate Specificity of the L-Type Amino Acid Transporter (LAT1). J.Med.Chem. 2018, 61(16):7358-7373.
Coultrap, S.J., Ashpole, N.M., Hudmon, A., Bayer, K.U., CaMKII in cerebral ischemia. Acta Pharmacol Sin, 2011, 32, 861-872.
Clarkson, A.N., Huang, B.S., Macisaac, S.E., Mody, I., Carmichael, S.T., Reducing excessive GABA-mediated tonic inhibition promotes functional recovery after stroke. Nature, 2010, 468, 305-309.
Gavande, N.; Johnston, G. A. R.; Hanrahan, J. R.; Chebib, M. Microwave-enhanced synthesis of 2,3,6-trisubstituted pyridazines: application to four-step synthesis of gabazine (SR-95531). Organic & Biomolecular Chemistry, 2010, 8, 4131-4136.
Guery, S.; Parrot, I.; Rival, Y.; Wermuth, C. G. Efficient one-step synthesis of 3-amino-6-arylpyridazines. Tetrahedron Letters 2001, 42, 2115-2117.
Hell, J. W. CaMKII: claiming center stage in postsynaptic function and organization. Neuron, 2014, 81, 249-265.
Iqbal, F.; Ellwood, R.; Mortensen, M.; Smart, T. G.; Baker, J. R. Synthesis and evaluation of highly potent GABAA receptor antagonists based on gabazine (SR-95531). Bioorganic & Medicinal Chemistry Letters, 2011, 21, 4252-4254.
Huang, Y.S., Guilleminault, C. Narcolepsy: action of two gamma-aminobutyric acid type B agonists, baclofen and sodium oxybate. Pediatr. Neurol., 2009, 41, 9-16.
Kool, M.J., Van De Bree, J.E., Bodde, H.E., Elgersma, Y., Van Woerden, G.M., The molecular, temporal and region-specific requirements of the beta isoform of Calcium/Calmodulin-dependent protein kinase type 2 (CAMK2B) in mouse locomotion. Sci. Rep. 2016, 6:26989, 1-12.
Krall, J.; Bavo, F.; Falk-Petersen, C. B.; Jensen, C. H.; Nielsen, J. O.; Tian, Y.; Anglani, V.; Kongstad, K. T.; Piilgaard, L.; Nielsen, B.; Gloriam, D. E.; Kehler, J.; Jensen, A. A.; Harpsøe, K.; Wellendorph, P.; Frølund, B., Discovery of 2-(Imidazo[1,2-b]pyridazin-2-yl)acetic Acid as a New Class of Ligands Selective for the γ-Hydroxybutyric Acid (GHB) High-Affinity Binding Sites. Journal of Medicinal Chemistry 2019, 62 (5), 2798-2813.
Petrignet, J.; Thiery, E.; Silpa, L.; Abarbri, M. Mild and direct access to 7-substituted-4-trifluoromethylpyrimido[1,2-b]pyridazin-2-one systems. Synthesis 2014, 46, 947-954.
Puris, E. et al. L-type amino acid transporter 1 utilizing prodrugs: How to achieve effective brain delivery and low systemic exposure of drugs. J. Control. Release 2017, 261, 93-104.
Rajalu, M., Fritzius, T., Adelfinger, L., Jacquier, V., Besseyrias, V., Gassmann, M., Bettler, B., Pharmacological characterization of GABAB receptor subtypes assembled with auxiliary KCTD subunits. Neuropharmacol., 2015, 88, 145-154.
Tabuchi, S., Tsunematsu, T., Black, S. W., Tominaga, M., Maruyama, M., Takagi, K., Conditional ablation of orexin/hypocretin neurons: a new mouse model for the study of narcolepsy and orexin system function. J. Neurosci., 2014, 34, 6495-509.
Thiesen, L., Kehler, J., Clausen, R.P., Frølund, B., Bundgaard, C., Wellendorph, P., In vitro and in vivo evidence for active brain uptake of the GHB analog HOCPCA by the monocarboxylate transporter subtype 1. J. Pharmacol. Exp.Ther. 2015, 354, 166-174.
Vogensen, S.B., Marek, A., Bay, T., Wellendorph, P., Kehler, J., Bundgaard, C., Frølund, B., Pedersen, M.H., Clausen, R.P. New synthesis and tritium labeling of a selective ligand for studying high-affinity γ-hydroxybutyrate (GHB) binding sites. J. Med. Chem. 2013, 56, 8201-8205.
Wellendorph, P., Høg, S., Greenwood, J.R., de Lichtenberg, A., Nielsen, B., Frølund, B., Brehm, L., Clausen, R.P., Bräuner-Osborne, H., Novel cyclic gamma-hydroxybutyrate (GHB) analogs with high affinity and stereoselectivity of binding to GHB sites in rat brain. J. Pharmacol. Exp. Ther. 2005, 315, 346-351.

## Claims

1. A compound of general formula I: 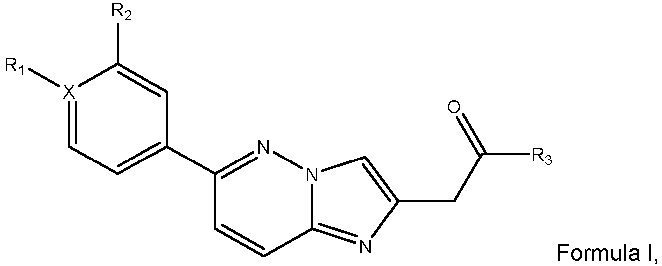
or a pharmaceutically acceptable salt thereof,
wherein:
X is C, R₁ and R₂ are different and selected from H, F, I, Cl, -OH, -NH₂, -NO₂, ethyl, propyl, isopropyl, butyl, isobutyl, *tert-butyl,* pentyl, hexyl wherein butyl, pentyl and hexyl are linear or branched, -O- C₁-C₆-alkyl, -O-benzyl (-O-Bn), wherein C₁-C₆-alkyl is linear or branched and wherein benzyl is unsubstituted or substituted with one or more halogen and/or one or more C₁-C₆-alkyl or -O-C₁-C₆-alkyl;
or
X is C, R₁ and R₂ are the same and selected from F, I, Cl, -OH, -NH₂, -NO₂, ethyl, propyl, isopropyl, butyl, isobutyl, *tert-butyl,* pentyl, hexyl wherein butyl, pentyl and hexyl are linear or branched, -O- C₁-C₆-alkyl, -O-benzyl (-O-Bn), wherein C₁-C₆-alkyl is linear or branched and wherein benzyl is unsubstituted or substituted with one or more halogen and/or one or more C₁-C₆-alkyl or -O-C₁-Cₑ-alkyl;
and
R₃ is selected from -OH, -NH₂, -O-C₁-C₆-alkyl, -C₁-C₆-alkyl-O-C(=O)-C₁-C₆-alkyl, -NH-C₁-C₆-alkyl, - O-aryl, O-substituted aryl, -NH-aryl, -NH-substituted aryl, wherein C₁-C₆-alkyl is linear or branched, aryl is unsubstituted or substituted with one or more amino acid residues to form a compound in formula II illustrated with a substituted aryl being represented by phenylalanine: 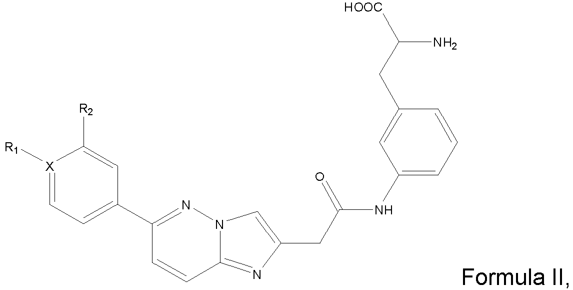
or a pharmaceutically acceptable salt thereof; provided that the compound is not one of the following, wherein
| X | R₁ | R₂ | R₃ |
|---|---|---|---|
| C | Cl | H | OH |
| C | H | Cl | OH |
| C | OMe | H | OH |
| C | H | OMe | OH |
| C | Cl | Cl | OH |
| C | OBn | H | OH |
| C | H | OBn | OH |
or hydrochloride or TFA salts of compounds mentioned in the table above.

2. The compound according to claim 1, wherein C₁-C₆-alkyl is selected from methyl, ethyl, propyl, isopropyl, butyl, isobutyl, *tert-*butyl*,* pentyl, hexyl; wherein butyl, pentyl and hexyl are linear or branched.

3. The compound according to any one of the preceding claims, wherein R₁ and R₂ are the same or different and selected from H, F, I, Cl, -O-C₁-C₆-alkyl and -O-Bn, and wherein R₃ and alkyl are according to any one of the preceding claims.

4. The compound according to any one of the preceding claims, wherein R₁ and R₂ are the same or different and selected from H, F, I, Cl, -O-C₁-C₆-alkyl and -O-Bn, and R₃ is selected from -OH, -O-C₁-C₆-alkyl, -C₁-C₆-alkyl-O-C(=O)-C₁-C₆-alkyl, and -NH-substituted aryl, wherein alkyl and aryl are according to any one of the preceding claims.

5. The compound according to any one of the preceding claims, wherein one of R₁ and R₂ is H and the other is selected from F, I, Cl, -O-C₁-C₆-alkyl and -O-Bn; or wherein R₁ and R₂ are the same or different and selected from F, I, Cl, -O-C₁-C₆-alkyl and -O-Bn, and R₃ is according to any one of the preceding claims.

6. The compound according to any one of claims 1-3, and 5, wherein R₃ is selected from -NH₂, -O-C₁-C₆-alkyl, -C₁-C₆-alkyl-O-C(=O)-C₁-C₆-alkyl, -NH-C₁-C₆-alkyl, -NH-aryl, -NH-substituted aryl, wherein C₁-C₆-alkyl is linear or branched, aryl is unsubstituted or substituted with one or more amino acid residues to form a compound in formula II illustrated with a substituted aryl being represented by phenylalanine: 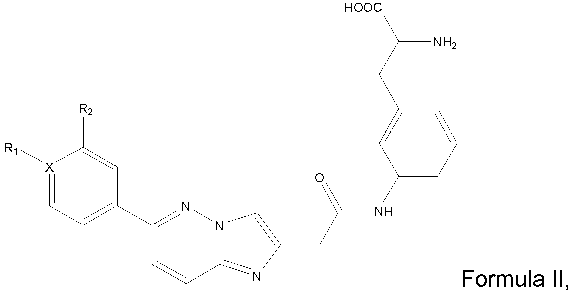 or a pharmaceutically acceptable salt thereof.

7. A compound of general formula I 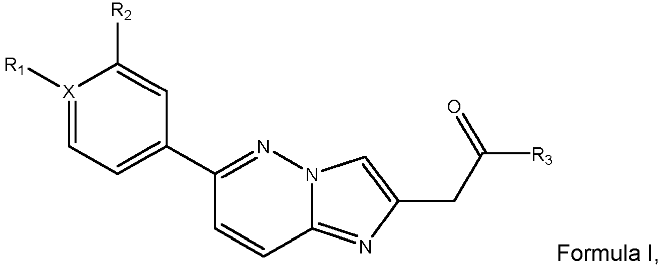
or a pharmaceutically acceptable salt thereof,
wherein X is C, R₁ and R₂ are the same or different and selected from H, halogen, -OH, -NH₂, - NO₂, -C₁-C₆-alkyl, -O-C₁-C₆-alkyl, O-benzyl (O-Bn), wherein C₁-C₆-alkyl is linear or branched and
wherein benzyl is unsubstituted or substituted with one or more halogen and/or one or more -C₁-C₆-alkyl or -O C₁-C₆-alkyl; and
R₃ is selected from -OH, -NH₂, -O-C₁-C₆-alkyl, -C₁-C₆-alkyl-O-C(=O)-C₁-C₆-alkyl, -NH-C₁-C₆-alkyl, - O-aryl, -O-substituted aryl -NH-aryl, -NH-substituted aryl, wherein C₁-C₆-alkyl is linear or branched,
aryl is unsubstituted or substituted with one or more amino acid residues to form a compound in formula II illustrated with a substituted aryl being represented by phenylalanine: 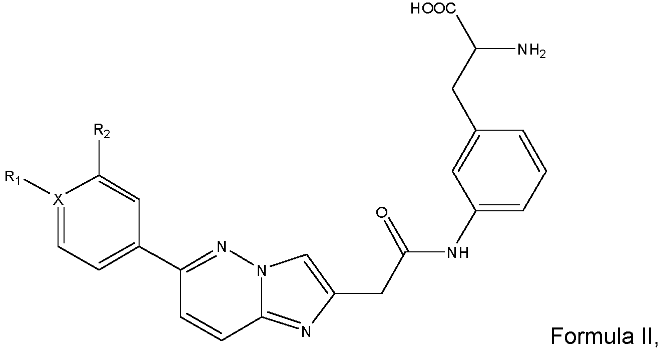
or a pharmaceutically acceptable salt thereof for use in medicine.

8. The compound according to any one of claims 1-6 or the compound for use according to claim 7, for use in a method of treating brain injuries, such as acute brain injuries, such as a brain injury selected from the group consisting of traumatic brain injury, stroke, subarachnoid haemorrhage, neonatal hypoxia-ischemia encephalopathy or associated in utero complications, haemodynamic shock with cardiac arrest, and global hypoperfusion during surgery or as a result from heart failure.

9. The compound according to any one of claims 1-6 or the compound for use according to claim 7, for use in a method of treating central hypersomnia, such as a central hypersomnia selected from the group consisting of idiopathic hypersomnia and recurrent hypersomnia including Klein-Levin syndrome and narcolepsy with cataplexy (narcolepsy type 1) and narcolepsy without cataplexy (narcolepsy type 2); or for use in a method of treating cataplexy.

10. The compound for use according to any one of claims 7-9, wherein halogen is selected from F, I, Cl and Br.

11. The compound for use according to any one of claims 7-10, wherein C₁-C₆-alkyl is selected from methyl, ethyl, propyl, isopropyl, butyl, isobutyl, *tert-*butyl*,* pentyl, hexyl; wherein butyl, pentyl and hexyl are linear or branched.

12. The compound for use according to any one of claims 7-11, wherein R₁ and R₂ are the same or different and selected from H, halogen, -O-C₁-C₆-alkyl and -O-Bn, and R₃, halogen and alkyl are according to any one of claims 7-11.

13. The compound for use according to any one of claims 7-12, wherein R₁ and R₂ are the same or different and selected from H, halogen, -O-C₁-C₆-alkyl and -O-Bn, and R₃ is selected from -OH, -O-C₁-C₆-alkyl, -C₁-C₆-alkyl-O-C(=O)-C₁-C₆-alkyl, and -NH-substituted aryl, wherein halogen, alkyl and aryl are according to any one of claims 7-12.

14. The compound for use according to any one of claims 7-13, wherein one of R₁ and R₂ is H and the other is selected from halogen, -O-C₁-C₆-alkyl and -O-Bn; or wherein R₁ and R₂ are the same or different and selected from halogen, -O-C₁-C₆-alkyl and -O-Bn, and R₃ is according to any one of claims 7-13.

15. The compound for use according to any one of claims 7-10 and 12-14, wherein R₃ is selected from -NH₂, -O-C₁-C₆-alkyl, -C₁-C₆-alkyl-O-C(=O)-C₁-C₆-alkyl, -NH-C₁-C₆-alkyl, -NH-aryl, -NH-substituted aryl, wherein C₁-C₆-alkyl is linear or branched, aryl is unsubstituted or substituted with one or more amino acid residues to form a compound in formula II illustrated with a substituted aryl being represented by phenylalanine: 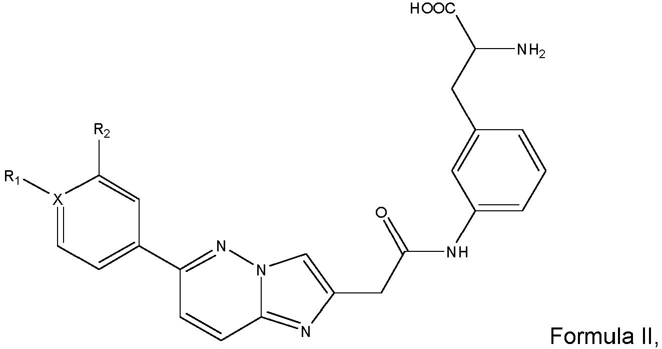 or a pharmaceutically acceptable salt thereof.

16. The compound for use according to any one of claims 7-14, where the compound has formula I, and wherein X, R₁, R₂, and R₃ are as set out in the table:
| X | R₁ | R₂ | R₃ |
|---|---|---|---|
| C | Cl | H | OH |
| C | H | Cl | OH |
| C | OMe | H | OH |
| C | H | OMe | OH |
| C | Cl | Cl | OH |
| C | OBn | H | OH |
| C | H | OBn | OH |
or pharmaceutically acceptable salts thereof.

17. The compound for use according to any one of claims 7-15 having one of the following formulas: 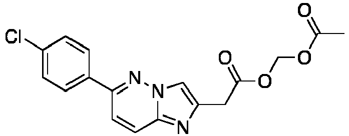 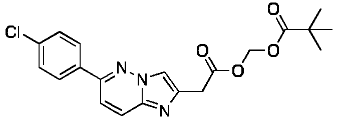 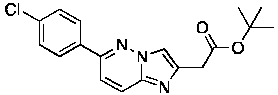 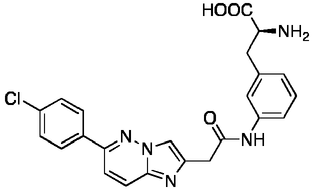 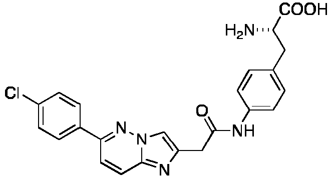 or a pharmaceutically acceptable salt thereof.

## Patentansprüche

1. Verbindung der allgemeinen Formel I: 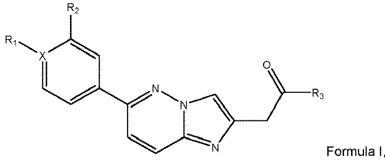
oder ein pharmazeutisch verträgliches Salz davon,
wobei:
X C ist, R₁ und R₂ verschieden sind und aus H, F, I, Cl, -OH, - NH₂, -NO₂, Ethyl, Propyl, Isopropyl, Butyl, Isobutyl, tert-Butyl, Pentyl, Hexyl ausgewählt sind, wobei Butyl, Pentyl und Hexyl linear oder verzweigt sind, -O- C₁-C₆-Alkyl, -O-Benzyl (-O-Bn),
wobei C₁-C₆-Alkyl linear oder verzweigt ist und wobei Benzyl unsubstituiert oder substituiert ist mit einem oder mehreren Halogenen und/oder einem oder mehreren C₁-C₆-Alkyl oder -O-C₁-C₆-Alkyl;
oder
X C ist, R₁ und R₂ gleich sind und aus F, I, Cl, -OH, -NH₂, -NO₂, Ethyl, Propyl, Isopropyl, Butyl, Isobutyl, tert-Butyl, Pentyl, Hexyl ausgewählt sind, wobei Butyl, Pentyl und Hexyl linear oder verzweigt sind, -O- C₁-C₆-Alkyl, -O-Benzyl (-O-Bn), wobei C₁-C₆-Alkyl linear oder verzweigt ist und wobei Benzyl unsubstituiert oder substituiert ist mit einem oder mehreren Halogenen und/oder einem oder mehreren C₁-C₆-Alkyl oder -O-C₁-C₆-Alkyl;
und
R₃ ist ausgewählt aus -OH, -NH₂, -O-C₁-C₆-Alkyl, -C₁-C₆-Alkyl-O-C(=O)-C₁-C₆-Alkyl, -NH-C₁-C₆-Alkyl, -O-Aryl, O-substituiertem Aryl, -NH-Aryl, -NH-substituiertem Aryl, wobei C₁-C₆-Alkyl linear oder verzweigt ist, Aryl unsubstituiert oder mit einem oder mehreren Aminosäureresten substituiert ist, um eine Verbindung der Formel II zu bilden, die mit einem durch Phenylalanin dargestellten substituierten Aryl veranschaulicht ist: 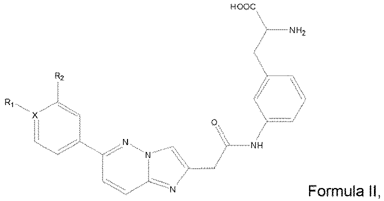
oder ein pharmazeutisch verträgliches Salz davon, vorausgesetzt, dass die Verbindung nicht eine der folgenden ist, wobei
| X | R₁ | R₂ | R₃ |
|---|---|---|---|
| C | Cl | H | OH |
| C | H | Cl | OH |
| C | OMe | H | OH |
| C | H | OMe | OH |
| C | Cl | Cl | OH |
| C | OBn | H | OH |
| C | H | OBn | OH |
oder Hydrochlorid- oder TFA-Salze der in der obigen Tabelle genannten Verbindungen.

2. Verbindung nach Anspruch 1, wobei C₁-C₆-Alkyl aus Methyl, Ethyl, Propyl, Isopropyl, Butyl, Isobutyl, *tert*-Butyl, Pentyl, Hexyl ausgewählt ist; wobei Butyl, Pentyl und Hexyl linear oder verzweigt sind.

3. Verbindung nach einem der vorhergehenden Ansprüche, wobei R₁ und R₂ gleich oder verschieden sind und aus H, F, I, Cl, -O-C₁-C₆-Alkyl und -O-Bn ausgewählt sind und wobei R₃ und Alkyl nach einem der vorhergehenden Ansprüche sind.

4. Verbindung nach einem der vorhergehenden Ansprüche, wobei R₁ und R₂ gleich oder verschieden sind und aus H, F, I, Cl, -O-C₁-C₆-Alkyl und -O-Bn ausgewählt sind und R₃ aus -OH, -O-C₁-C₆-Alkyl, -C₁-C₆-Alkyl-O-C(=O)-C₁-C₆-Alkyl und -NH-substituiertem Aryl ausgewählt ist, wobei Alkyl und Aryl nach einem der vorhergehenden Ansprüche sind.

5. Verbindung nach einem der vorhergehenden Ansprüche, wobei einer von R₁ und R₂ H ist und der andere aus F, I, Cl, -O-C₁-C₆-Alkyl und -O-Bn ausgewählt ist; oder wobei R₁ und R₂ gleich oder verschieden sind und aus F, I, Cl, -O-C₁-C₆-Alkyl und -O-Bn ausgewählt sind und R₃ nach einem der vorhergehenden Ansprüche ist.

6. Verbindung nach einem der Ansprüche 1 bis 3 und 5, wobei R₃ aus -NH₂, -O-C₁-C₆-Alkyl, -C₁-C₆-Alkyl-O-C(=O)-C₁-C₆-Alkyl, -NH-C₁-C₆-Alkyl, -NH-Aryl, -NH-substituiertem Aryl ausgewählt ist, wobei C₁-C₆-Alkyl linear oder verzweigt ist, Aryl unsubstituiert oder mit einem oder mehreren Aminosäureresten substituiert ist, um eine Verbindung der Formel II zu bilden, die mit einem durch Phenylalanin dargestellten substituierten Aryl veranschaulicht ist: 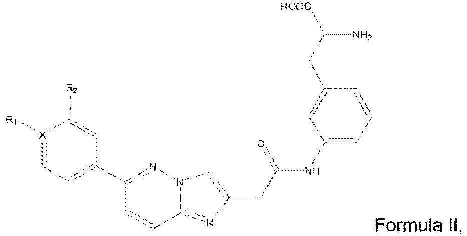 oder ein pharmazeutisch verträgliches Salz davon.

7. Verbindung der allgemeinen Formel I 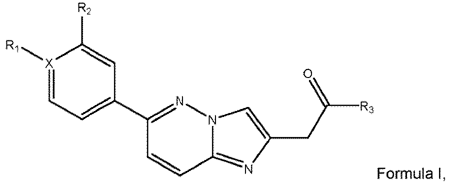
oder ein pharmazeutisch verträgliches Salz davon,
wobei X C ist, R₁ und R₂ gleich oder verschieden sind und aus H, Halogen, -OH, -NH₂, -NO₂, -C₁-C₆-Alkyl, -O-C₁-C₆-Alkyl, O-Benzyl (O-Bn) ausgewählt sind, wobei C₁-C₆-Alkyl linear oder verzweigt ist und wobei Benzyl unsubstituiert oder substituiert ist mit einem oder mehreren Halogenen und/oder einem oder mehreren -C₁-C₆-Alkyl oder -O C₁-C₆-Alkyl; und
R₃ aus -OH, -NH₂, -O-C₁-C₆-Alkyl, -C₁-C₆-Alkyl-O-C(=O)-C₁-C₆-Alkyl, -NH-C₁-C₆-Alkyl, -O-Aryl, -O-substituiertem Aryl, -NH-Aryl, -NH-substituiertem Aryl ausgewählt ist, wobei C₁-C₆-Alkyl linear oder verzweigt ist, Aryl unsubstituiert oder mit einem oder mehreren Aminosäureresten substituiert ist, um eine Verbindung der Formel II zu bilden, die mit einem durch Phenylalanin dargestellten substituierten Aryl veranschaulicht ist: 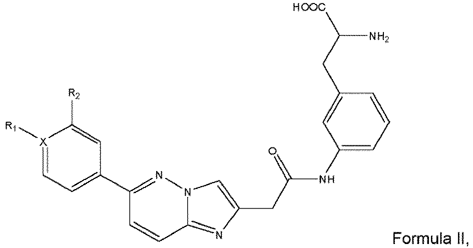 oder ein pharmazeutisch verträgliches Salz davon zur Verwendung in der Medizin.

8. Verbindung nach einem der Ansprüche 1 bis 6 oder Verbindung zur Verwendung nach Anspruch 7 zur Verwendung bei einem Verfahren zur Behandlung von Hirnverletzungen, wie akuten Hirnverletzungen, wie einer Hirnverletzung, ausgewählt aus der Gruppe, bestehend aus traumatischer Hirnverletzung, Schlaganfall, Subarachnoidalblutung, neonataler Hypoxie-Ischämie-Enzephalopathie oder damit verbundenen Komplikationen in utero, hämodynamischem Schock mit Herzstillstand und globaler Hypoperfusion während einer Operation oder als Folge von Herzversagen.

9. Verbindung nach einem der Ansprüche 1 bis 6 oder Verbindung zur Verwendung nach Anspruch 7 zur Verwendung bei einem Verfahren zur Behandlung von zentraler Hypersomnie, wie etwa einer zentralen Hypersomnie, ausgewählt aus der Gruppe, bestehend aus idiopathischer Hypersomnie und wiederkehrender Hypersomnie, einschließlich Klein-Levin-Syndrom und Narkolepsie mit Kataplexie (Narkolepsie Typ 1) und Narkolepsie ohne Kataplexie (Narkolepsie Typ 2); oder zur Verwendung bei einem Verfahren zur Behandlung von Kataplexie.

10. Verbindung zur Verwendung nach einem der Ansprüche 7 bis 9, wobei Halogen aus F, I, Cl und Br ausgewählt ist.

11. Verbindung zur Verwendung nach einem der Ansprüche 7 bis 10, wobei C₁-C₆-Alkyl aus Methyl, Ethyl, Propyl, Isopropyl, Butyl, Isobutyl, *tert*-Butyl, Pentyl, Hexyl ausgewählt ist; wobei Butyl, Pentyl und Hexyl linear oder verzweigt sind.

12. Verbindung zur Verwendung nach einem der Ansprüche 7 bis 11, wobei R₁ und R₂ gleich oder verschieden sind und aus H, Halogen, -O-C₁-C₆-Alkyl und -O-Bn ausgewählt sind und R₃, Halogen und Alkyl nach einem der Ansprüche 7 bis 11 sind.

13. Verbindung zur Verwendung nach einem der Ansprüche 7 bis 12, wobei R₁ und R₂ gleich oder verschieden sind und aus H, Halogen, -O-C₁-C₆-Alkyl und -O-Bn ausgewählt sind und R₃ aus -OH, -O-C₁-C₆-Alkyl, -C₁-C₆-Alkyl-O-C(=O)-C₁-C₆-Alkyl und -NH-substituiertem Aryl ausgewählt ist, wobei Halogen, Alkyl und Aryl nach einem der Ansprüche 7 bis 12 sind.

14. Verbindung zur Verwendung nach einem der Ansprüche 7 bis 13, wobei einer von R₁ und R₂ H ist und der andere aus Halogen, -O-C₁-C₆-Alkyl und -O-Bn ausgewählt ist; oder wobei R₁ und R₂ gleich oder verschieden sind und aus Halogen, -O-C₁-C₆-Alkyl und -O-Bn ausgewählt sind, und R₃ nach einem der Ansprüche 7 bis 13 ist.

15. Verbindung zur Verwendung nach einem der Ansprüche 7 bis 10 und 12 bis 14, wobei R₃ aus -NH₂, -O-C₁-C₆-Alkyl, -C₁-C₆-Alkyl-O-C(=O)-C₁-C₆-Alkyl, -NH-C₁-C₆-Alkyl, -NH-Aryl, -NH-substituiertem Aryl ausgewählt ist, wobei C₁-C₆-Alkyl linear oder verzweigt ist, Aryl unsubstituiert oder mit einem oder mehreren Aminosäureresten substituiert ist, um eine Verbindung der Formel II zu bilden, die mit einem durch Phenylalanin dargestellten substituierten Aryl veranschaulicht ist: 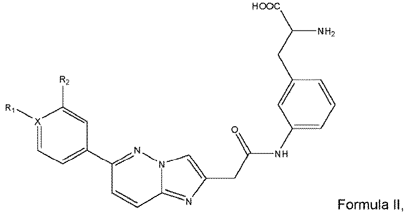 oder ein pharmazeutisch verträgliches Salz davon.

16. Verbindung zur Verwendung nach einem der Ansprüche 7 bis 14, wobei die Verbindung die Formel I hat, und wobei X, R₁, R₂ und R₃ wie in der Tabelle angegeben sind:
| X | R₁ | R₂ | R₃ |
|---|---|---|---|
| C | Cl | H | OH |
| C | H | Cl | OH |
| C | OMe | H | OH |
| C | H | OMe | OH |
| C | Cl | Cl | OH |
| C | OBn | H | OH |
| C | H | OBn | OH |
oder pharmazeutisch verträgliche Salze davon.

17. Verbindung zur Verwendung nach einem der Ansprüche 7 bis 15 mit einer der folgenden Formeln: 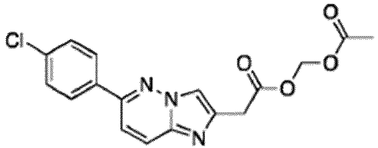 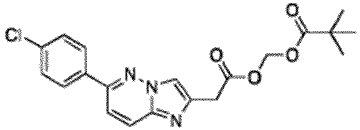 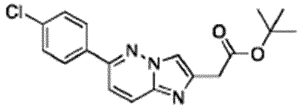 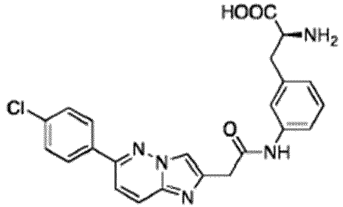 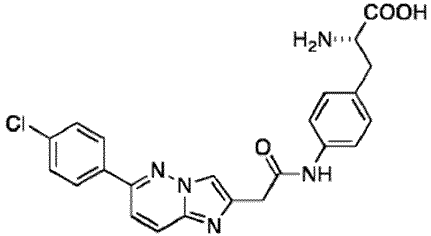 oder ein pharmazeutisch verträgliches Salz davon.

## Revendications

1. Composé de formule générale I : 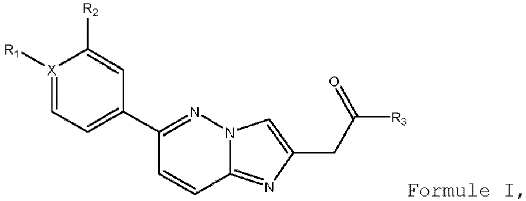
ou un sel pharmaceutiquement acceptable de celui-ci,
dans lequel :
X est C, R₁ et R₂ sont différents et choisis parmi H, F, I, Cl, -OH, -NH₂, -NO₂, éthyle, propyle, isopropyle, butyle, isobutyle, tert-butyle, pentyle, hexyle dans lequel butyle, pentyle et hexyle sont linéaires ou ramifiés, -O-alkyle en C₁-C₆, -O-benzyle (-O-Bn), dans lequel alkyle en C₁-C₆ est linéaire ou ramifié et dans lequel benzyle est non substitué ou substitué par un ou plusieurs halogènes et/ou un ou plusieurs alkyle en C₁-C₆ ou -O-alkyle en C₁-C₆ ;
ou
X est C, R₁ et R₂ sont identiques et choisis parmi F, I, Cl, - OH, -NH₂, -NO₂, éthyle, propyle, isopropyle, butyle, isobutyle, tert-butyle, pentyle, hexyle dans lequel butyle, pentyle et
hexyle sont linéaires ou ramifiés, -O-alkyle en C₁-C₆, -O-benzyle (-O-Bn), dans lequel alkyle en C₁-C₆ est linéaire ou ramifié et dans lequel benzyle est non substitué ou substitué par un ou plusieurs halogènes et/ou un ou plusieurs alkyle en C₁-C₆ ou -O-alkyle en C₁-C₆ ;
et
R₃ est choisi parmi -OH, -NH₂, -O-alkyle en C₁-C₆, alkyle en C₁-C₆-O-C(=O)-alkyle en C₁-C₆, -NH-alkyle en C₁-C₆, -O-aryle, O-aryle substitué, -NH-aryle, -NH-aryle substitué, dans lequel alkyle en C₁-C₆ est linéaire ou ramifié, aryle est non substitué ou substitué par un ou plusieurs résidus d'acides aminés pour former un composé de formule II illustrée par un aryle substitué étant représenté par la phénylalanine : 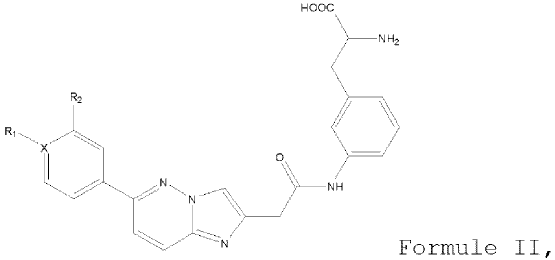
ou un sel pharmaceutiquement acceptable de celui-ci ; à condition que le composé ne soit pas l'un des suivants, dans lequel
| X | R₁ | R₂ | R₃ |
|---|---|---|---|
| C | Cl | H | OH |
| C | H | Cl | OH |
| C | OMe | H | OH |
| C | H | OMe | OH |
| C | Cl | Cl | OH |
| C | OBn | H | OH |
| C | H | OBn | OH |
ou chlorhydrate ou sels de TFA des composés mentionnés dans le tableau ci-dessus.

2. Composé selon la revendication 1, dans lequel l'alkyle en C₁-C₆ est choisi parmi méthyle, éthyle, propyle, isopropyle, butyle, isobutyle, tert-butyle, pentyle, hexyle ; dans lequel butyle, pentyle et hexyle sont linéaires ou ramifiés.

3. Composé selon l'une quelconque des revendications précédentes, dans lequel R₁ et R₂ sont identiques ou différents et choisis parmi H, F, I, Cl, -O-alkyle en C₁-C₆ et -O-Bn, et dans lequel R₃ et alkyle sont selon l'une quelconque des revendications précédentes.

4. Composé selon l'une quelconque des revendications précédentes, dans lequel R₁ et R₂ sont identiques ou différents et choisis parmi H, F, I, Cl, -O-alkyle en C₁-C₆ et -O-Bn, et R₃ est choisi parmi -OH, -O-alkyle en C₁-C₆, alkyle en C₁-C₆-O-C(=O)-alkyle en C₁-C₆, et -NH-aryle substitué, dans lequel alkyle et aryle sont selon l'une quelconque des revendications précédentes.

5. Composé selon l'une quelconque des revendications précédentes, dans lequel l'un de R₁ et de R₂ est H et l'autre est choisi parmi F, I, Cl, -O-alkyle en C₁-C₆ et -O-Bn ; ou dans lequel R₁ et R₂ sont identiques ou différents et choisis parmi F, I, Cl, -O-alkyle en C₁-C₆ et -O-Bn, et R₃ est selon l'une quelconque des revendications précédentes.

6. Composé selon l'une quelconque des revendications 1 à 3 et 5, dans lequel R₃ est choisi parmi -NH₂, -O-alkyle en C₁-C₆, alkyle en C₁-C₆-O-C(=O)-alkyle en C₁-C₆, -NH-alkyle en C₁-C₆, -NH-aryle, -NH-aryle substitué, dans lequel alkyle en C₁-C₆ est linéaire ou ramifié, aryle est non substitué ou substitué par un ou plusieurs résidus d'acides aminés pour former un composé de formule II illustrée par un aryle substitué étant représenté par la phénylalanine : 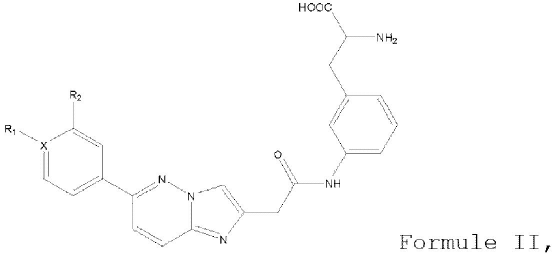 ou un sel pharmaceutiquement acceptable de celui-ci.

7. Composé de formule générale I 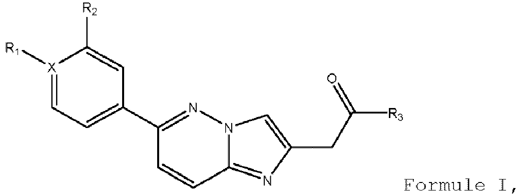
ou un sel pharmaceutiquement acceptable de celui-ci,
dans lequel X est C, R₁ et R₂ sont identiques ou différents et choisis parmi H, halogène, -OH, -NH₂, -NO₂, alkyle en C₁-C₆, -O-alkyle en C₁-C₆, O-benzyle (O-Bn), dans lequel alkyle en C₁-C₆ est linéaire ou ramifié et dans lequel benzyle est non substitué ou substitué par un ou plusieurs halogènes et/ou un ou plusieurs alkyle en C₁-C₆ ou -O-alkyle en C₁-C₆ ; et
R₃ est choisi parmi -OH, -NH₂, -O-alkyle en C₁-C₆, alkyle en C₁-C₆-O-C(=O)-alkyle en C₁-C₆, -NH-alkyle en C₁-C₆, -O-aryle, O-aryle substitué, -NH-aryle, -NH-aryle substitué, dans lequel alkyle en C₁-C₆ est linéaire ou ramifié, aryle est non substitué ou substitué par un ou plusieurs résidus d'acides aminés pour former un composé de formule II illustrée par un aryle substitué étant représenté par la phénylalanine : 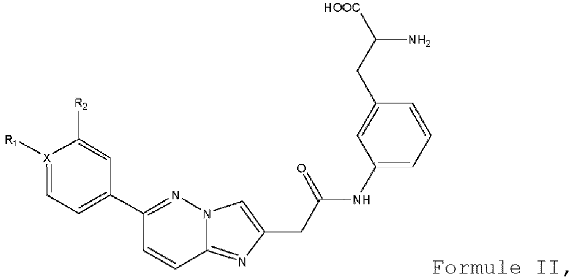
ou un sel pharmaceutiquement acceptable de celui-ci pour une utilisation en médecine.

8. Composé selon l'une quelconque des revendications 1 à 6 ou composé pour une utilisation selon la revendication 7, pour une utilisation dans un procédé de traitement de lésions cérébrales, telles que des lésions cérébrales aiguës, telles qu'une lésion cérébrale choisie dans un groupe composé d'une lésion cérébrale traumatique, d'un accident vasculaire cérébral, d'une hémorragie sous-arachnoïdienne, d'une encéphalopathie hypoxo-ischémique néonatale ou de complications in utero associées, d'un choc hémodynamique avec arrêt cardiaque et d'une hypoperfusion globale pendant une intervention chirurgicale ou résultant d'une insuffisance cardiaque.

9. Composé selon l'une quelconque des revendications 1 à 6 ou composé pour une utilisation selon la revendication 7, pour une utilisation dans un procédé de traitement de l'hypersomnie centrale, telle qu'une hypersomnie centrale choisie dans un groupe composé de l'hypersomnie idiopathique et de l'hypersomnie récurrente incluant le syndrome de Klein-Levin et la narcolepsie avec cataplexie (narcolepsie de type 1) et la narcolepsie sans cataplexie (narcolepsie de type 2) ; ou pour une utilisation dans un procédé de traitement de la cataplexie.

10. Composé pour une utilisation selon l'une quelconque des revendications 7 à 9, dans lequel l'halogène est choisi parmi F, I, Cl et Br.

11. Composé pour une utilisation selon l'une quelconque des revendications 7 à 10, dans lequel l'alkyle en C₁-C₆ est choisi parmi méthyle, éthyle, propyle, isopropyle, butyle, isobutyle, tert-butyle, pentyle, hexyle ; dans lequel butyle, pentyle et hexyle sont linéaires ou ramifiés.

12. Composé pour une utilisation selon l'une quelconque des revendications 7 à 11, dans lequel R₁ et R₂ sont identiques ou différents et choisis parmi H, halogène, -O-alkyle en C₁-C₆ et - O-Bn, et R₃, halogène et alkyle sont selon l'une quelconque des revendications 7 à 11.

13. Composé pour une utilisation selon l'une quelconque des revendications 7 à 12, dans lequel R₁ et R₂ sont identiques ou différents et choisis parmi H, halogène, -O-alkyle en C₁-C₆ et - O-Bn, et R₃ est choisi parmi -OH, -O-alkyle en C₁-C₆, alkyle en C₁-C₆-O-C(=O)-alkyle en C₁-C₆, et -NH-aryle substitué, dans lequel halogène, alkyle et aryle sont selon l'une quelconque des revendications 7 à 12.

14. Composé pour une utilisation selon l'une quelconque des revendications 7 à 13, dans lequel l'un de R₁ et de R₂ est H et l'autre est choisi parmi halogène, -O-alkyle en C₁-C₆ et -O-Bn ; ou dans lequel R₁ et R₂ sont identiques ou différents et choisis parmi halogène, -O-alkyle en C₁-C₆ et -O-Bn, et R₃ est selon l'une quelconque des revendications 7 à 13.

15. Composé pour une utilisation selon l'une quelconque des revendications 7 à 10 et 12 à 14, dans lequel R₃ est choisi parmi -NH₂, -O-alkyle en C₁-C₆, alkyle en C₁-C₆-O-C(=O)-alkyle en C₁-C₆, -NH-alkyle en C₁-C₆, -NH-aryle, -NH-aryle substitué, dans lequel alkyle en C₁-C₆ est linéaire ou ramifié, aryle est non substitué ou substitué par un ou plusieurs résidus d'acides aminés pour former un composé de formule II illustrée par un aryle substitué étant représenté par la phénylalanine : 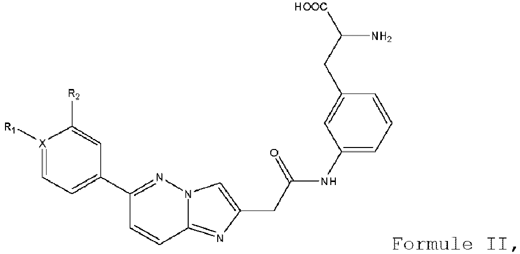 ou un sel pharmaceutiquement acceptable de celui-ci.

16. Composé pour une utilisation selon l'une quelconque des revendications 7 à 14, dans lequel le composé a la formule I, et dans laquelle X, R₁, R₂ et R₃ sont tels qu'indiqués dans le tableau :
| X | R₁ | R₂ | R₃ |
|---|---|---|---|
| C | Cl | H | OH |
| C | H | Cl | OH |
| C | OMe | H | OH |
| C | H | OMe | OH |
| C | Cl | Cl | OH |
| C | OBn | H | OH |
| C | H | OBn | OH |
ou des sels pharmaceutiquement acceptables de celui-ci.

17. Composé pour une utilisation selon l'une quelconque des revendications 7 à 15 ayant l'une des formules suivantes : 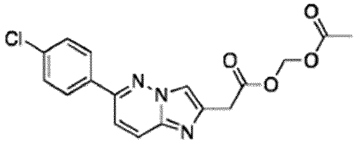 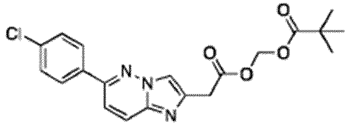 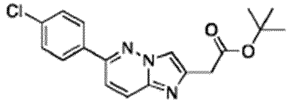 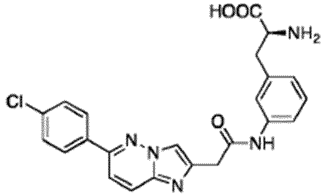 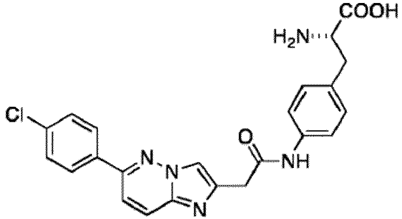 ou un sel pharmaceutiquement acceptable de celui-ci.
